# EUROPEAN PATENT SPECIFICATION

(11) **EP 0 932 418 B1**
(45) Date of publication and mention of the grant of the patent: **05.12.2007**
(21) Application number: 97943290.3
(22) Date of filing: 04.09.1997
(51) Int. Cl.: A61K 48/00

(54) **METHOD FOR RECOMBINANT ADENO-ASSOCIATED VIRUS-DIRECTED GENE THERAPY**
VERFAHEREN ZUR DURCH REKOMBINANTE ADENO-ASSOZIIERTE VIRUS-GERICHTETE GENTHERAPIE
METHODE DE THERAPIE GENIQUE BASEE SUR DES VIRUS ADENO-ASSOCIES DE RECOMBINAISON

(30) Priority: 06.09.1996 US 708188; 10.10.1996 US 729061
(43) Date of publication of application: 04.08.1999
(60) Divisional application: 06006222.1
(73) Proprietor: THE TRUSTEES OF THE UNIVERSITY OF PENNSYLVANIA, Philadelphia, Pennsylvania 19104-3147 (US)
(72) Inventor: WILSON, James, M., Gladwyne, PA 19035 (US); FISHER, Krishna, J., New Orleans, LA 70115 (US)
(74) Representative: Roques, Sarah Elizabeth
(86) International application number: PCT/US1997/015692
(87) International publication number: WO 1998/009657

(56) References cited:
- WO-A-95/34670
- WO-A-97/12050
- WO-A-97/26337
- US-A- 5 436 146
- COOVERT D D ET AL: "GENE THERAPY FOR MUSCLE DISEASES" CURRENT OPINION IN NEUROLOGY, vol. 7, no. 5, October 1994, pages 463-470, XP000673519
- SAMULSKI R J ET AL: "HELPER-FREE STOCKS OF RECOMBINANT ADENO-ASSOCIATED VIRUSESS NORMAL INTEGRATION DOES NOT REQUIRE VIRAL GENE EXPRESSION" JOURNAL OF VIROLOGY, vol. 63, no. 9, 1 September 1989, pages 3822-3828, XP000283159
- HERZOG ET AL: "STABLE GENE TRANSFER AND EXPRESSION OF HUMAN BLOOD COAGULATION FACTOR IX AFTER INTRAMUSCULAR INJECTION OF RECOMBINANT ADENO-ASSOCIATED VIRUS" PROCEEDINGS OF THE NATIONAL ACADEMY OF SCIENCES,USA, vol. 94, May 1997, pages 5804-5809, XP002054071
- FISHER ET AL: "RECOMBINANT ADENO-ASSOCIATED VIRUS FOR MUSCLE DIRECTED GENE THERAPY" NATURE MEDICINE, vol. 3, no. 3, March 1997, pages 306-312, XP002054072
- KESSLER ET AL: "GENE DELIVERY TO SKELETAL MUSCLE RESULTS IN SUSTAINED EXPRESSION AND SYSTEMIC DELIVERY OF A THERAPEUTIC PROTEIN" PROCEEDINGS OF THE NATIONAL ACADEMY OF SCIENCES,USA, vol. 93, November 1996, pages 14082-14087, XP002054073

## Description

This work was supported by the National Institutes of Health Grant No. DK47757. The U. S. Government has certain rights in this invention.

### Background of the Invention

Adeno-associated virus (AAV) is a replication-deficient parvovirus, the genome of which is about 4.6 kb in length, including 145 nucleotide inverted terminal repeats (ITRs). The single-stranded DNA genome of AAV contains genes responsible for replication (rep) and formation of virions (cap).

When this nonpathogenic human virus infects a human cell, the viral genome integrates into chromosome 19 resulting in latent infection of the cell. Production of infectious virus and replication of the virus does not occur unless the cell is coinfected with a lytic helper virus such as adenovirus or herpes virus. Upon infection with a helper virus, the AAV provirus is rescued and amplified, and both AAV and helper virus are produced.

AAV possesses unique features that make it attractive as a vector for delivering foreign DNA to cells. Various groups have studied the potential use of AAV in the treatment of disease states.

Studies of recombinant AAV (rAAV) *in vitro* have been disappointing because of low frequencies of transduction; incubation of cells with rAAV in the absence of contaminating wild-type AAV or helper adenovirus is associated with little recombinant gene expressiont [D. Russell et al, Proc. Natl. Acad. Sci. USA, 91:8915-8919 (1994); I. Alexander et al, J. Virol., 68:8282-8287 (1994); D. Russell et al, Proc. Natl. Acad. Sci. USA, 92:5719-5723 (1995); K. Fisher et al, J. Virol., 70:520532 (1996); and F. Ferrari et al, J. Virol., 70:3227-3234(1996)]. Furthermore, integration is inefficient and not directed to chromosome 19 when rep is absent [S. Kumar et al, J. Mol. Biol., 222:45-57 (1991)]. AAV transduction is substantially enhanced in the presence of adenovirus because the single-stranded rAAV genome is converted to a non-integrated, double-stranded intermediate which is transcriptionally active [K. Fisher et al, J. Virol., 70:520-532 (1996); and F. Ferrari et al, J. Virol., 70:3227-3234 (1996)]. Adenovirus augments rAAV transduction through the expression of the early gene product E4 ORF6 [K. Fisher et al, J. Virol., 70:520-532 (1996); and F. Ferrari et al, J. Virol., 70:3227-3234 (1996)].

The performance of rAAV as a vector for *in vivo* models of gene therapy has been mixed. The most promising results have been in the central nervous system, where stable transduction has been achieved in postmitotic cells [M. Kaplitt et al, Nat. Genet., 8:148-154 (1994)]. Incubation of bone marrow cells *ex vivo* with rAAV results in some transduction, although stable and efficient hematopoietic engraftment has not been demonstrated in transplant models [J. Miller et al, Proc. Natl. Acad. Sci. USA, 91:10183-10187 (1994); G. Podsakoff et al, J. Virol., 68:5656-5666 (1994); and C. Walsh et al, J. Clin. Invest., 94:1440-1448 (1994)]. Administration of rAAV into the airway or the blood leads to gene transfer into lung epithelial cells [K. Fisher et al, J. Virol., 70:520-532 (1996); and T. Flotte et al, Proc. Natl. Acad. Sci. USA, 90:10613-10617 (1993)] and hepatocytes [K. Fisher et al, J. Virol., 70:520-532 (1996)] respectively; however, transgene expression has been found to be low unless adenovirus is present [K. Fisher et al, J. Virol., 70:520-532 (1996)].

What is needed is a method of improving rAAV-mediated gene transfer.

### Summary of the invention

The invention provides use of a recombinant adeno-associated virus (rAAV) comprising a heterologous nucleic acid sequence encoding a product operably linked to sequences which control expression thereof in a cell for the manufacture of a medicament for the treatment of a chronic disorder, wherein the medicament is in a form adapted for delivery of the AAV to a muscle cell which expresses the product and the rAAV is purified from contaminating helper adenovirus so that the product is expressed in the absence of a destructive immune response to the product, and wherein the medicament is adapted for delivery by repeated administration, and the product is a neoantigen.

The present invention relates to improving expression of a selected gene delivered to an animal via recombinant AAV. The invention relates to introducing a recombinant AAV vector comprising a desired transgene into a muscle cell in the absence of a helper virus. The vector may be administered into cardiac, smooth, or, preferably, skeletal muscle.

In one preferred embodiment, the rAAV-delivered transgene encodes a secretable and/or diffusable product which is therapeutically useful. In this embodiment, the transgene product may have therapeutic effect on sites at a distance from the delivery site. In another embodiment, the transgene encodes a non-secretable product (e.g., a dystrophin polypeptide) for which delivery to the muscle is desired (e.g., for treatment of muscular dystrophy).

In another aspect, the present invention relates to treating an animal with hemophilia. The invention relates to administering into the muscle of the animal a recombinant adeno-associated virus comprising the gene encoding factor IX and sequences which regulate expression of the gene.

In yet another aspect, the invention relates to treating an animal with atherosclerosis. The invention relates to administering into the muscle of the animal a recombinant adeno-associated virus comprising the gene encoding ApoE and regulatory sequences capable of expressing said gene.

Other aspects and advantages of the present invention are described further in the following detailed description of the preferred embodiments thereof.

### Brief Description of the Drawings

Fig. 1 is a schematic illustration showing the linear arrangement of AAV.CMVLacZ (4883 bp). The relevant elements include AAV ITRs (solid black boxes), CMV promoter (hatched arrow), SV40 intron and polyadenylation signal (open boxes), and lacZ DNA (shaded box). The location of a cDNA probe that can be used to detect the internal BamHI fragment, as well as full-length vector, is also shown.
Fig. 2A is a schematic illustration of the linear arrangement of AAV.CMVLacZ concatomer. The relevant landmarks include AAV ITRs (hatched boxes), CMV enhancer/promoter (solid black arrow), SV40 intron and polyadenylation signal (open boxes), and lacZ cDNA (shaded box). AAV.CMVLacZ monomer is shown joined according to a direct end-to-end ligation mechanism (labeled j) at the ITRs. Therefore, in the cartoon two copies of the AAV ITR are present at the junction.
Fig. 2B is a schematic illustration showing an amplified view of the junction domain. Relevant landmarks are as indicated in Fig. 3A above. Horizontal arrows indicate the location and direction of PCR primers used to amplify across the provirus junction. Primer 005 is a sense-strand primer. Primers 013 and 017 are antisense-strand primers.
Fig. 3 is a schematic illustration showing the predicted PCR product assuming a direct end-to-end tandem ligation of monomer AAV.CMVLacZ genomes. Two complete ITRs (shaded box) with their respective "FLOP" and "FLIP" orientation are shown at the junction (labeled j). The CMV promoter (solid black box) and polyadenylation signal (open box) are also indicated. The PCR cloning site in pCRII is flanked by EcoRI sites as shown. The location of three SnaBI sites positioned within the PCR product are also shown. Primers 005 and 013 also indicated.
Fig. 4A, in conjunction with Figs. 4B-4G, illustrates the structure of PCR products that map to the head-to-tail junction of AAV.CMVLacZ concatomers of Fig. 4A. Fig. 4A shows the predicted PCR product assuming a direct end-to-end tandem ligation of monomer AAV.CMVLacZgenomes. Two complete ITRs (shaded box) with their respective "FLOP" and "FLIP" orientation are shown at the junction (labeled j). The CMV promoter (solid black box) and polyadenylation signal (open box) are also indicated.
Fig. 4B illustrates the structure of the PCR product from Clone 3.
Fig. 4C illustrates the structure of the PCR product from Clone 8. Clone 8 is nearly identical in size to clone 3, but contains a different rearrangement of the ITR junction.
Fig. 4D illustrates the structure of the PCR product from Clone 5.
Fig. 4E illustrates the structure of the PCR product from Clone 2.
Fig. 4F illustrates the structure of the PCR product from Clone 6.
Fig. 4G illustrates the structure of the PCR product from Clone 7.
Fig. 5A characterizes the activation of cytotoxic T lymphocytes directed against adenoviral antigen as well as *lacZ.* This is an analysis of lymphocytes harvested from Group 1 of Example 5.
Fig. 5B characterizes the activation of cytotoxic T lymphocytes directed against adenoviral antigen as well as *lacZ*. This is an analysis of lymphocytes harvested from Group 2 of Example 5.
Fig. 5C characterizes the activation of cytotoxic T lymphocytes directed against adenoviral antigen as well as *lacZ.* This is an analysis of lymphocytes harvested from Group 3 of Example 5.
Fig. 6A shows the activation of T lymphocytes in response to different antigens including β-galactosidase, purified AAV, or adenovirus, for each of Groups 1-4 of Example 5. Activation is demonstrated by the secretion of IFN-γ representing the TH1 subset of T cells.
Fig. 6B shows the activation of T lymphocytes in response to different antigens including β-galactosidase, purified AAV, or adenovirus, for each of Groups 1-4 of Example 5. Activation is demonstrated by the secretion of IL-10 representing the TH2 subset of T cells.
Fig. 7A illustrates results from an enzyme linked immunosorbent assay (ELISA), showing the development of antibodies directed against β-galactosidase in the various groups of example 5.
Fig. 7B illustrates results from an enzyme linked immunosorbent assay (ELISA), showing the development of antibodies directed against adenovirus type 5 in the various groups of Example 5.
Fig. 8 is a graph of plasma concentration of hF.IX in C57BL/6 mice as a function of time following IM injection of 2X10¹¹ rAAV-hF.IX vector genomes/animal (n=4).
Fig. 9 is a graph showing circulating antibody against human F.IX as a result of im injection of rAAV-hF.IX in C57BL/6 mice. The time course of anti-hF.IX antibody concentration in plasma after injection with 2X10¹¹ vector genomes/animal (n=3) was determined by ELISA using mouse MAb anti-hF.IX [Boehringer Mannheim] as a standard. Each line represents an individual animal.
Fig. 10 illustrates plasma concentration of hF.IX in three mice as a function of time post-injection. Each symbol represents a different animal. The fourth animal in this experiment died 5 weeks post-injection following traumatic phlebotomy.
Fig. 11 is a graph illustrating plasma concentration of hF.IX in four Rag-1 mice as a function of time post-injection with rAAV-hF.IX. Each symbol represents a different animal.
Fig. 12 is a schematic diagram of a head-to-tail concatamer of the rAAV present in transduced cells.
Fig. 13 is a schematic diagram illustrating the construction of AV.CMVApoE.

### Detailed Description of the Invention

The present invention relates to adeno-associated virus (AAV) mediated muscle-directed gene transfer which provides high level and stable transgene expression in the absence of helper virus or exogenous helper molecules, Particularly, this involves introducing a recombinant AAV carrying a desired transgene into a muscle cell. Desirably, the rAAV vector is injected directly into cardiac, skeletal, or smooth muscle and the transgene encodes a secreted and/or diffusable therapeutic product, such as a polypeptide or an RNA molecule. However, the invention is similarly useful for administration of nucleic acids encoding non-secreted, therapeutically useful products.

Particularly, the inventors have discovered that intramuscular injection of helper-free purified rAAV (i.e., rAAV which is substantially free of contamination with adenovirus or wild-type AAV) leads to highly efficient transduction of muscle fibers leading to stable and prolonged transgene expression. By helper-free is further meant that the AAV is in the substantial absence of helper virus or other exogenous helper molecules (i.e., helper molecules not native to or normally present in muscle cells). This is accomplished without significant inflammation or activation of immune responses to the transgene product, despite the fact that the product may be a neoantigen.

The stability of transgene expression produced according to the invention is particularly impressive. Without wishing to be bound by theory, this stability is believed to be due to the highly inefficient chromosomal integration of AAV proviral DNA in muscle cells in the absence of helper virus or other exogenous helper molecules. Several observations support this hypothesis. As described in the examples below, analysis of Hirt extracts failed to detect a double-stranded episomal form of the viral genome. Southern analysis of total cellular DNA revealed a discrete band when digested with an enzyme that has two cleavage sites within the AAV vector, whereas no discrete band was observed when the same DNA was digested with an enzyme that does not have sites within the viral genome.

Further DNA analysis focused on the formation of concatamers and their structure. Previous studies of lytic AAV infections have shown that the episomal replication of rAAV proceeds through head-to-head or tail-to-tail concatamers, while latent infections that result in proviral integration are established as head-to-tail tandem arrays [K.I. Berns, Microbiol. Rev., 54:316-329 (1990); J.-D. Tratschin et al, Mol. Cell. Biol., 5:3251-3260 (1985); N. Muzcyzska, Current Topics in Microbiology and Immunology, 158:97-129 (1993); S.K. McLauglin et la, J. Virol., 62:1963-1973 (1988)]. The data set forth herein demonstrate that muscle cells transduced with rAAV vectors according to this invention contain concatamers consisting of head-to-tail tandem arrays with variable deletions of both ITRs, consistent with a transduction mechanism involving integration of rAAV provirus.

Sequence analysis of the junctions created by rAAV concatamers indicated consistent but variable deletions of both ITRs. Fluorescent in situ hybridization (FISH) analysis was consistent with single integration sites in approximately 1 in 20 nuclei, while Southern analysis indicated an average of 1 proviral genome per diploid genome of the muscle fiber. Together these findings indicate that the average concatamers minimally comprises ten proviral genomes.

Another advantage of the invention is the surprising absence of inflammation upon administration of therapeutic doses of vector. For example, C57BL/6 mice injected with a lacZ AAV vector failed to mount a humoral immune response to *E.coli* β-galactosidase despite the fact that vibrant anti-B-galactosidase antibodies were elicited in these animals when a lacZ adenoviral vector was injected into skeletal muscle. Thus, when the helper-free AAV vector was used according to the invention, immune responses to the transgene were modulated. This contrasts sharply with prior art methods of gene transfer, such as those which employ naked plasmid DNA [J.A. Wolff et al, Science, 247:1465-1468 (1990)] or adenovirus-mediated gene therapy [S.K. Tripathy et al, Nat, Med., 2:545-550 (1996)], which typically elicit strong immune responses to the transgene. Thus, the invention provides a significant advantage over other gene delivery systems, particularly with respect to the treatment of chronic disorders that may require repeated administrations.

### I. The Recombinant AAV

A recombinant AAV vector carrying a selected transgene is used in the invention. In addition to the transgene, the vector further contains regulatory sequences which control expression of the transgene in a host cell, e.g., a muscle cell.

Many rAAV vectors are known to those of skill in the art and the invention is not limited to any particular rAAV vector. For example, suitable AAV vectors and methods of producing same are described in U. S. Patent No. 5,252,479; U. S. Patent No. 5,139,941; International Patent Application No. WO94/13788; and International Patent Application No. WO93/24641. One particularly desired vector is described below.

### A. The AAV Sequences

Currently, a preferred rAAV is deleted of all viral open reading frames (ORFs) and retains only the cis-acting 5' and 3' inverted terminal repeat (ITR) sequences [See, e.g., B. J. Carter, in "Handbook of Parvoviruses", ed., P. Tijsser, CRC Press, pp.155-168 (1990)]. Thus, the rep and cap polypeptide encoding sequences are deleted. The AAV ITR sequences are about 143 bp in length. While it is preferred that substantially the entire 5' and 3' sequences which comprise the ITRs are used in the vectors, the skilled artisan will understand that some degree of minor modification of these sequences is permissible. The ability to modify these ITR sequences while retaining their biological functions is within the skill of the art. See, e.g., texts such as Sambrook et al, "Molecular Cloning. A Laboratory Manual.", 2d edit., Cold Spring Harbor Laboratory, New York (1989).

The AAV ITR sequences may be obtained from any known AAV, including presently identified human AAV types. The selection of the AAV type is not anticipated to limit the invention. A variety of AAV types, including types 1-4, are available from the American Type Culture Collection or available by request from a variety of commercial and institutional sources. Similarly, AAVs known to infect other animals may also be employed in the vector used in the invention. In the examples set forth herein, an AAV-2 is used for convenience. Specifically, the 5' and 3' AAV ITR sequences from AAV-2 flank a selected transgene sequence and associated regulatory elements, as described below.

### B. The Transgene

The transgene sequence contained within the rAAV vector is a nucleic acid sequence, heterologous to the AAV sequence, which encodes an RNA or polypeptide of interest. The transgene is operatively linked to regulatory components in a manner which permits transgene expression in muscle cells.

The composition of the transgene sequence will depend upon the use to which the resulting vector will be put. For example, one type of transgene sequence includes a reporter sequence, which upon expression produces a detectable signal. Such reporter sequences include without limitation an *E*. *coli* beta-galactosidase (*LacZ*) cDNA, an alkaline phosphatase gene and a green fluorescent protein gene. These sequences, when associated with regulatory elements which drive their expression, provide signals detectable by conventional means, e.g., ultraviolet wavelength absorbance, visible color change, etc. Expression of such transgenes may be used in methods for cell quantitation, cell identification and the like.

A more preferred transgene sequence includes a therapeutic gene which encodes a desired gene product. These therapeutic nucleic acid sequences typically encode products which, when administered to a patient *in vivo* or *ex vivo,* are able to replace or correct an inherited or non-inherited genetic defect or treat an epigenetic disorder or disease.

Delivery of the transgene to the muscle cells, is particularly well suited for use in connection with secreted therapeutic proteins, such as factor IX, useful in treatment of hemophilia, or apolipoprotein (Apo) E, useful in treatment of atherosclerosis. However, other therapeutic gene products, particularly those which are secreted, may be readily selected by the skilled artisan. Examples of genes encoding secreted and/or diffusable products, include, without limitation, cytokines, growth factors, hormones, differentiation factors, and the like, e.g., β-interferon (β-IFN), erythropoietin (epo), insulin, growth hormone (GH), and parathyroid hormone (PTH). These genes are useful for treatment of a variety of conditions, including multiple sclerosis and cancer (β-IFN), anemia (epo), diabetes (insulin), small stature (GH), and osteoporosis (PTH). The invention is also useful for delivery of genes encoding non-secreted products to the muscle. For example, the invention is anticipated to be useful in treatment of muscular dystrophies, by enabling delivery of a dystrophin gene [see, e.g., C. C. Lee et al, Nature, 349:334-336 (1991)] via a rAAV according to the method of the invention. The selection of the transgene is not considered to be a limitation of this invention, as such selection is within the knowledge of the skilled artisan.

### C. Regulatory Elements of the Vector

In addition to the AAV ITR sequences and the transgene, the vector also includes regulatory elements necessary to drive expression of the transgene in transduced muscle cells. Thus the vector desirably contains a selected promoter and enhancer (if desired) which is operatively linked to the transgene and located, with the transgene, between the AAV ITR sequences of the vector.

Selection of the promoter and, if desired, the enhancer, is a routine matter and is not a limitation of the vector itself. Useful promoters may be constitutive promoters or regulated (inducible) promoters, which will enable controlled expression of the transgene. For example, a desirable promoter is that of the cytomegalovirus immediate early promoter/enhancer [see, e.g., Boshart et al, Cell, 41:521-530 (1985)]. Other desirable promoters include, without limitation, the Rous sarcoma virus LTR promoter/enhancer and the inducible mouse metallothienien promoter. Still other promoter/enhancer sequences may be selected by one of skill in the art.

The vectors will also desirably contain nucleic acid sequences which affect transcription or translation of the transgene including sequences providing signals required for efficient polyadenylation of the transcript and introns with functional splice donor and acceptor sites. A common poly-A sequence which is employed in the exemplary vectors of this invention is that derived from the papovavirus SV-40. The poly-A sequence generally is inserted into the vector following the transgene sequences and before the 3' AAV ITR sequence. A common intron sequence is also derived from SV-40, and is referred to as the SV-40 T intron sequence. Selection of these and other elements desirable to control or enhance gene expression are conventional and many such sequences are known to those of skill in the art [see, e.g., Sambrook et al, and references cited therein].

The combination of the transgene, promoter/enhancer and the other regulatory elements are referred to as a "minigene" for ease of reference herein. As above stated, the minigene is flanked by the 5' and 3' AAV ITR sequences. Provided with the teachings of this invention, the design of such a minigene can be readily accomplished by the skilled artisan.

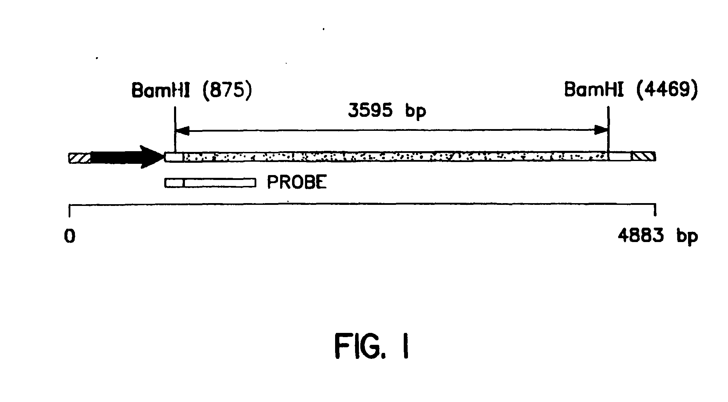

An example of a rAAV, i.e., AAV.CMVLacZ, and its use in the method of the invention is provided in the examples below. As illustrated in Fig. 1, this exemplary rAAV contains a 5'AAV ITR, a CMV promoter, an SV-40 intron, a LacZ transgene, an SV-40 poly-A sequence and a 3'AAV ITR. However, as stated above, the invention is not limited to use of any particular rAAV.

### D. Production of rAAV

The sequences employed in the construction of the rAAV used with the invention may be obtained from commercial or academic sources based on previously published and described materials. These materials may also be obtained from an individual patient or generated and selected using standard recombinant molecular cloning techniques known and practiced by those skilled in the art. Any modification of existing nucleic acid sequences used in the production of the rAAV vectors, including sequence deletions, insertions, and other mutations may also be generated using standard techniques.

Assembly of the rAAV, including the sequences of AAV, the transgene and other vector elements, may be accomplished using conventional techniques. One particularly desirable technique is described in K. J. Fisher et al, J. Virol., 70(1):520-532 (January, 1996). However, other suitable techniques include cDNA cloning such as those described in texts [Sambrook et al, cited above], use of overlapping oligonucleotide sequences of the AAV genome, polymerase chain reaction, and any suitable method which provides the desired nucleotide sequence. Where appropriate, standard transfection and co-transfection techniques are employed to propagate the rAAV viruses in the presence of helper viruses, e.g., adenoviruses deleted for E1 using techniques such as CaPO₄ transfection techniques, and may be readily selected by the skilled artisan. Other conventional methods which may be employed in this invention include homologous recombination of AAV viral genomes, plaquing of viruses in agar overlay, methods of measuring signal generation, and the like.

Desirably, the rAAV produced are purified to remove any contaminating adenovirus or wild-type AAV. A particularly desirable purification scheme is described in K. J. Fisher et al, J. Virol., 70(1):520-532 (January, 1996). However, one of skill in the art can readily select other appropriate purification means.

### II. Therapeutic Applications

Once a rAAV containing a desired transgene is obtained, the vector is administered directly into an animal's muscle. One advantage of the invention is that muscle is particularly well suited as a site for production of secreted therapeutic products, such as factor IX or apolipoprotein (Apo) E, among others. Alternatively, the invention is used to deliver a non-secreted gene product to the muscle cells.

The rAAV vectors of the present invention may be administered to a patient, preferably suspended in a biologically compatible solution or pharmaceutically acceptable carrier or delivery vehicle. A suitable vehicle includes sterile saline. Other aqueous and non-aqueous isotonic sterile injection solutions and aqueous and non-aqueous sterile suspensions known to be pharmaceutically acceptable carriers and well known to those of skill in the art may be employed for this purpose.

The rAAV vectors of this invention are administered in sufficient amounts to provide for integration and expression of the selected transgene such that a therapeutic benefit may be obtained without undue adverse effects and with medically acceptable physiological effects which can be determined by those skilled in the medical arts. In a preferred embodiment, the rAAV are injected directly into cardiac, skeletal, or smooth muscle. One of skill in the art will also appreciate that other methods of administration, e.g., intravenous or intraarterial injection may also be utilized in the method of the invention so long as the rAAV is targeted to the muscle cells.

Dosages of the rAAV vector will depend primarily on factors such as the condition being treated, the selected transgene, the age, weight and health of the patient, and may thus vary among patients. A therapeutically effective dose of the rAAV of the present invention is believed to be in the range of from about 1 to about 50 ml of saline solution containing concentrations of from about 1 x 10⁸ to 1 x 10¹¹ particles/ml rAAV vector of the present invention. Desirably, each dose contains at least 10⁹ particles rAAV. A more preferred human dosage is about 1-20 ml saline solution at the above concentrations. The levels of expression of the selected transgene can be monitored by bioassay to determine the route, dose or frequency of administration. Administration of the rAAV may be repeated as needed.

The examples set forth below illustrate the preferred methods for preparing the vectors and performing the methods of the invention. These examples are illustrative only and do not limit the scope of the invention.

### Example 1 - Production of AAV.CMVLacZ

A recombinant AAV (rAAV) was generated in which the rep and cap genes were replaced with a minigene expressing *E. coli* β-galactosidase under the control of a CMV promoter (AAV.CMVlacZ). AAV.CMVlacZ was produced by using the cis-acting plasmid pAAV.CMVlacZ, which was derived from psub201 [R. Samulski et al, J. Virol., 61(10):3096-3101 (1987)]. Briefly, the plasmid was transfected into 293 cells infected with E1-deleted adenovirus [K.J. Fisher et al, J. Virol., 70:520-532 (1996)] and AAV *rep* and *cap* functions were provided by a transacting plasmid pAAV/Ad [R. Samulski et al, J. Virol., 63:3822-3826 (1989)]. Production lots of AAV.CMVLacZ vector were titered according to genome copies/ml as described [Fisher et al, J. Virol., 70:520-532 (1996)].

The 5'-to-3' organization of the AAV.CMVLacZ genome (4883 bp), includes:
the 5' AAV ITR (bp 1-173) obtained by PCR using pAV2 [C. A. Laughlin et al, Gene, 23: 65-73 (1983)] as template [nucleotide numbers 365-538 of SEQ ID NO: 1];
a CMV immediate early enhancer/promoter [Boshart et al, Cell, 41:521-530 (1985); nucleotide numbers 563-1157 of SEQ ID NO: 1],
an SV40 intron (nucleotide numbers 1178-1179 of SEQ ID NO: 1),
an *E. coli* lacZ cDNA (nucleotide numbers 1356 - 4827 of SEQ ID NO: 1),
an SV40 polyadenylation signal (a 237 Bam HI-BclI restriction fragment containing the cleavage/poly-A signals from both the early and late transcription units; nucleotide numbers 4839 - 5037 of SEQ ID NO: 1) and
the 3' AAV ITR, obtained from pAV2 as a SnaBI-BgIII fragment (nucleotide numbers 5053 - 5221 of SEQ ID NO: 1) .

With reference to Fig. 1, two Bam HI sites are present in the double-stranded vector sequence. The first is located in the SV40 intron at bp position 875 and the second lies between the lacZ DNA and the SV40 polyadenylation signal at bp position 4469. Therefore, digestion of the double-stranded sequence with BamHI releases a fragment of 3595 bp in length. The location of a cDNA probe that can be used to detect the internal BamHI fragment, as well as full-length vector, is also shown.

The rAAV.CMV*lacZ* virus was purified using standard techniques [see, e.g., K. F. Kozarsky et al, J. Biol. Chem., 269:13695-13702 (1994)]. Stocks of rAAV used in the following examples were tested to ensure the absence of replication competent wild-type AAV and helper E1-deleted adenovirus as follows.

293 cells were seeded in chamber slides and co-infected with wild-type adenovirus and an aliquot of the rAAV vector stock. Twenty hours post-infection, cells were fixed and incubated with a mouse monoclonal antibody against AAV capsid proteins (American Research Products). Antigen-antibody complex was detected with a FITC-conjugated secondary antibody. A positive signal was scored as one infectious AAV unit. Contaminating helper adenovirus was measured by infecting 293 cells with an aliquot of the rAAV vector stock and staining for alkaline phosphatase reporter expression. The helper adenovirus is El-deleted and contains a human placenta alkaline phosphatase cDNA under the transcriptional control of the CMV promoter. Replication-competent wild-type AAV or adenovirus helper was not detected in the highly purified preparations of rAAV.

### Example 2 - rAAV Stably Transduces Skeletal Muscle in Vivo

rAAV was administered with and without an E2a-deleted adenovirus, which was intended to enhance transduction. Animal procedures were approved by the Institutional Animal Care and Use Committee (IACUC) of the Wistar Institute.

Briefly, five week old female C57BL/6 mice (Jackson Laboratories, Bar Harbor, Maine) were anesthetized with an intraperitoneal injection of ketamine (70 mg/kg) and xylazine (10 mg/kg) and a subsequent 1 cm lower extremity incision was made. Samples of rAAV.CMVLacZ (1x10⁹ vector genomes) in 25 *µ*l of HEPES-Buffered-Saline (HBS, pH 7.8) or rAAV.CMVlacZ supplemented with an adenovirus E2a mutant d1802 [S. A. Rice and D. L. Klessig, J. Virol., 56:767-778 (1985)] (5x10¹⁰ A₂₆₀ particles, 1x10⁸ pfu) just prior to injection, were injected into the tibialis anterior muscle of each leg using a Hamilton syringe. Incisions were closed with 4-0 Vicryl suture. To analyze transgene expression, animals were necropsied at various time points post-injection and injected muscle was excised with a scalpel. Tissue was placed on a drop of OTC embedding compound, snap-frozen in liquid nitrogen-cooled isopentane for seven seconds, and immediately transferred to liquid nitrogen. Analysis of tissue at each time point represented a minimum of 6 injection sites (i.e., bilateral sampling from at least 3 animals).

For histochemical analysis, frozen muscle was segmented laterally into two equal halves, generating a cross-section face of the tissue. Both halves of the tissue were serially sectioned (6 *µ*m). For X-gal histochemistry, sections were fixed in a freshly prepared 0.5% glutaraldehyde solution in PBS and stained for β-galactosidase activity as described [K.J. Fisher et al, J. Virol., 70:520-532 (1996)]. Sections were counterstained in neutral red solution and mounted.

When adenovirus was used as a helper for the rAAV, X-gal histochemistry analysis revealed high level transduction of muscle fibers by day 17 associated with substantial inflammation. Surprisingly, however, animals that received rAAV in the absence of adenovirus helper demonstrated levels of transduction that exceeded those found in the presence of adenovirus. These high levels have persisted without apparent diminution for 180 days.

### Example 3 - rAAV Genome Integrates with High Efficiency as Truncated Head-to-Tail Concatamers

In order to characterize the molecular state of the stabilized rAAV genome, Southern blot analysis of DNA from skeletal muscle harvested from mice injected as above was performed. Models in which the rAAV genome persists either as an episomal double-stranded genome such as those formed during lytic infection, or as an integrated provirus resembling latent infection were considered.

Briefly, low molecular weight DNA (Hirt) (see Part A below) and high molecular weight genomic DNA (see Part B below) was isolated from mouse muscle at selected time points. DNA samples were resolved on a 1% agarose gel and electrophoretically transferred to a nylon membrane (Hybond-N, Amersham). The blot was hybridized with a ³²P-dCTP random-primer-labeled restriction fragment isolated from the lacZ cDNA.

### A. Detection of Episomal Double-Stranded Genome

To detect nonintegrated forms of the rAAV genome, Hirt extracts of transduced muscle DNA were analyzed by hybridization with a ³²P-labeled cDNA that maps to the probe sequence shown in Fig. 1. The Hirt DNA samples (15 *µ*l, equivalent to 15 mg tissue) were extracted from muscle harvested on day 8, 17, 30, and 64 post-injection.

DNA from a cultured cell line infected with rAAV in the presence of adenovirus was analyzed. The analysis of Hirt extracts from that cell line demonstrated the presence of both single-stranded and monomeric double-stranded forms of the virus. However, Hirt extracts of muscle transduced with rAAV alone demonstrated the single-stranded genome by day 8 that diminished to undetectable levels by day 64. Double-stranded forms of rAAV were never detected in the Hirt extracts even when the filters were over-exposed. This indicates that the single-stranded rAAV genome is efficiently transferred into cells of skeletal muscle; however, it is not converted to transcriptionally active episomal forms.

### B. Detection and Characterization of Integrated Proviral DNA

To detect integrated proviral DNA, additional hybridization studies were performed with total cellular DNA harvested from transduced skeletal muscle 64 days post-infection. Genomic DNA (10 *µ*g, equivalent to 18 *µ*g tissue) was digested with BamHI or HindIII, a restriction enzyme that does not cut proviral DNA. As expected, HindIII digestion resulted in a smear after gel fractionation and hybridization to a virus specific probe. However, when genomic DNA was digested with BamHI, which cuts twice within the provirus, a discrete band of the predicted size of 3.6 kb was detected at an abundance of approximately 1 proviral genome/diploid host cell genome.

The structure of the integrated provirus was characterized using PCR analysis to delineate the potential mechanisms of persistence. Previous studies of wild type and rAAV have suggested different pathways of DNA replication in the lytic and latent phases of the viral life cycle. Specifically, in the presence of helper virus, AAV replicates to form dimeric replicative intermediates by a mechanism that results in the synthesis of head-to-head or tail-to-tail concatamers. This contrasts with latent infections where the integrated proviral genome is characterized by head-to-tail genomic arrays.

Genomic DNA from skeletal muscle was subjected to PCR analysis to amplify junctions between AAV genomic concatamers. A PCR method to detect integrated rAAV was developed, based on data indicating that integrated forms of rAAV are typically found as head-to-tail concatomers. Specifically, oligonucleotide primers were synthesized to allow selective PCR amplification across head-to-tail junctions of two monomers of the AAV.CMVLacZ genome. The sense-strand primer 005 (5'-ATAAGCTGCAATAAACAAGT-3'; SEQ ID NO: 4) mapped to bp position 4584-4603 of the SV40 polyadenylation signal domain. The antisense-strand primer 013 (5'-CATGGTAATAGCGATGACTA-3'; SEQ ID NO:2) mapped to bp position 497-478 of the CMV promoter, while the antisense-strand primer 017 (5'-GCTCTGCTTATATAGACCTC-3'; SEQ ID NO:3) mapped to bp position 700-680 of the CMV promoter. If the ITRs are retained intact, oligos 005 + 013 [SEQ ID NO:2] should amplify a 797 bp fragment while oligos 005 and 017 [SEQ ID NO: 3] should amplify a 1000 bp fragment. It is important to emphasize that the predicted PCR product sizes are based on the assumption that provirus junctions contain two ITR copies. Amplification across a junction that has fewer than two copies will therefore generate a PCR product that is proportionally smaller in size.

PCR reactions were performed using 100 ng genomic DNA template and primer concentrations of 0.5 µM. The thermocycle profile was 94° C 1 min, 52° C 1 min, and 72° C 1 min 30 sec for 35 cycles; the 94° C denaturation step of the first cycle was 2 min, while the 72° C extension step of the last cycle was 10 min. PCR products were analyzed by agarose gel electrophoresis.

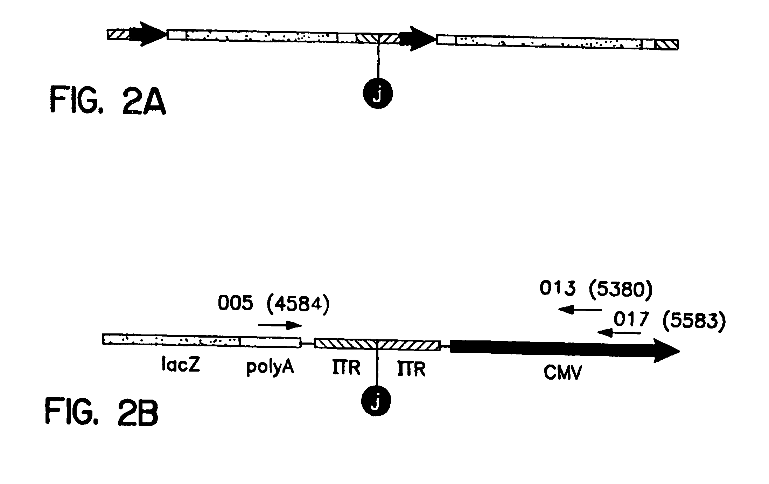

PCR reactions were conducted on genomic DNA isolated from AAV.CMVLacZ transduced muscle harvested at day 64 post-infection, as described above. Genomic DNA from muscle injected with Hepes buffered saline (HBS) was used as a negative PCR control. No amplification products were detected when primers were used that should span a head-to-head or tail-to-tail junction (data not shown). However, when DNA from AAV.CMVlacZ transduced muscle was analyzed with oligonucleotides 005 and 013 and 005 and 017, a smear was detected consistent with a heterogenous population of head-to-tail concatamers (Figs. 2A and 2B).

PCR reactions were also conducted with genomic DNA from cell lines that contain integrated AAV.CMVLacZ. The provirus structure of these clones has been determined by Southern blot analysis. Three cell lines (10-3.AV5, 10-3.AV6, and 10-3.AV18) each of which contain at least two monomer copies of integrated AAV.CMVLacZ arranged head-to-tail were identified. Based on the size of the PCR products (a 720 bp product using primer set 005-013, and a 930 bp product using primer set 005-017), two clones 10-3.AV5 and 10-3.AV6 likely contain 1.5 copies of AAV ITR at the junction. Another clone 10-3.AV18 contains a large deletion that encompasses the AAV ITRs producing a 320 bp product using primer set 005-013 and a 500 bp product using primer set 005-017. Another cell line 10-3.AV9 contains a single monomer copy of integrated AAV.CMVLacZ according to Southern blotting, and appears to be confirmed by the absence of a PCR product.

Thus, analysis of DNA from rAAV infected cell lines selected for stable transduction revealed distinct bands smaller than that predicted for an intact head-to-tail concatamer.

### D. Structural Analysis

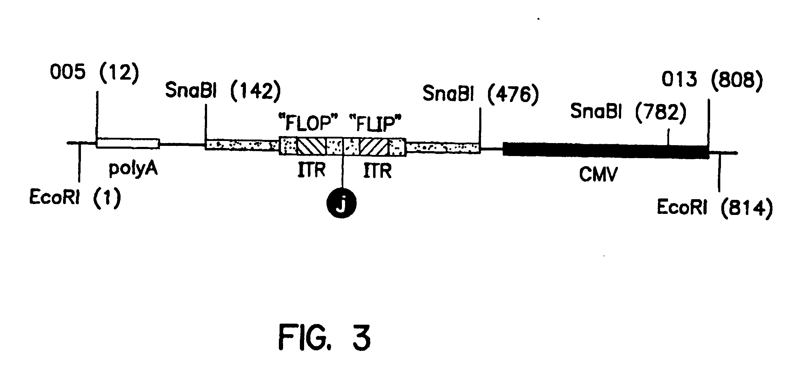

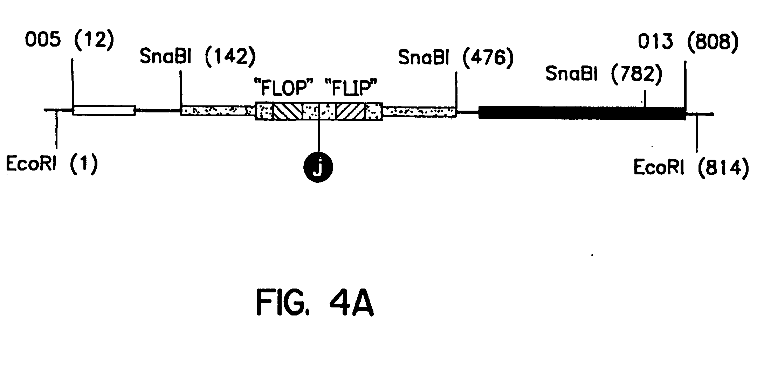

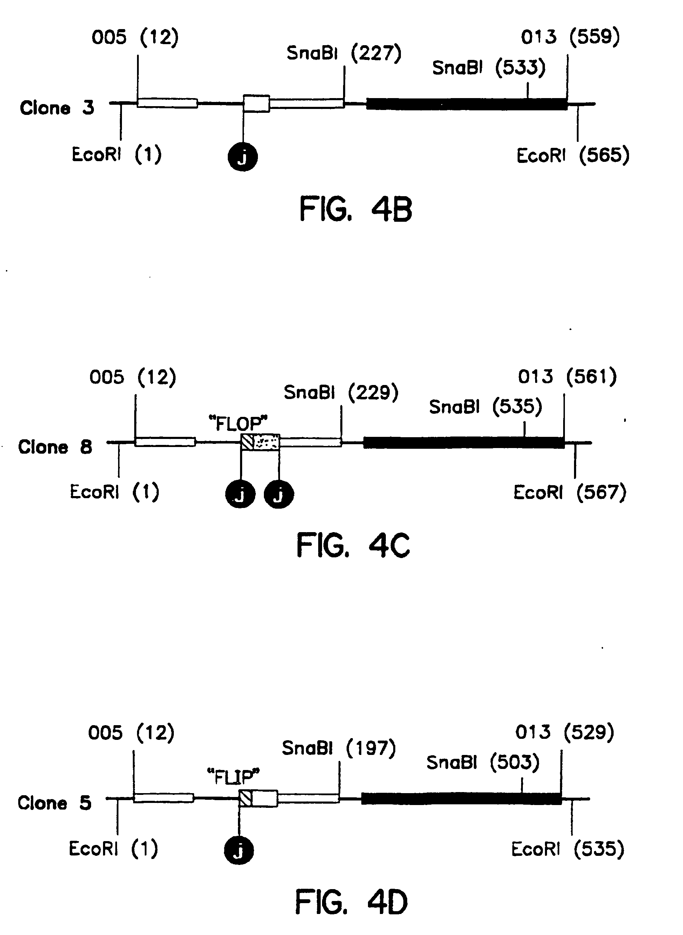

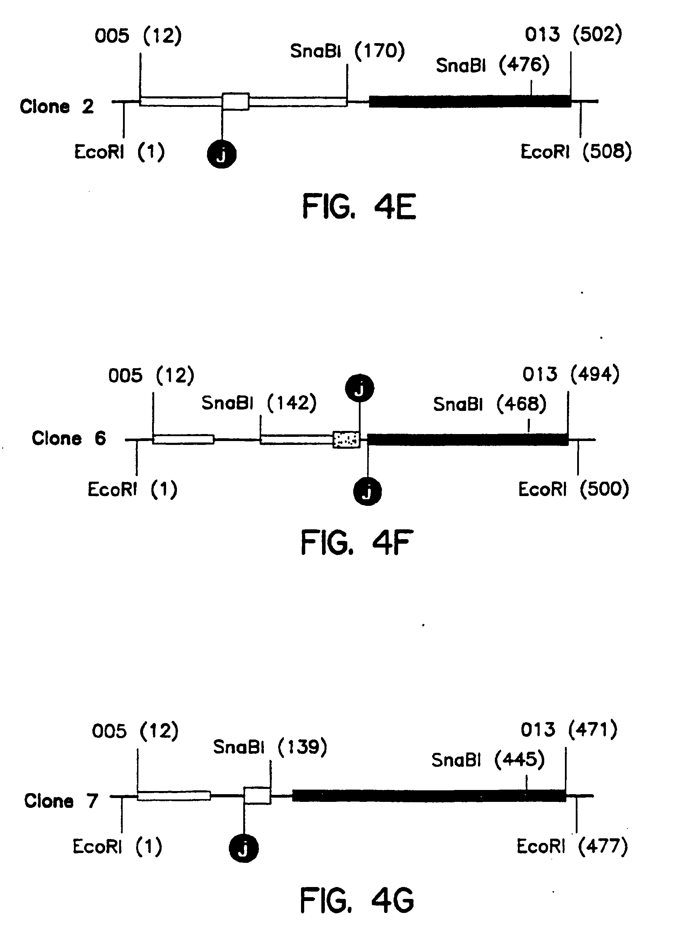

Detailed structural analyses of the proviral junctions recovered from skeletal muscle DNA was performed by subcloning from the PCR reaction (Fig. 3) followed by restriction analysis (Figs. 4A-4G). Particularly, PCR products from one of the muscle samples BL.11 obtained as described above were directly ligated into commercially available plasmid pCRII in which the insert was flanked by EcoRI sites. Commercially available competent bacterial strain TOP10 F' were transformed with the ligation reactions. In effect, this procedure results in a plasmid library of PCR products. The library was plated at a density to give well isolated colonies and screened by overlaying with a nylon membrane and hybridizing with a ³²P-labeled fragment corresponding to the CMV promoter/enhancer. Putative positive clones were grown overnight in small-scale cultures (2 ml).

Plasmid DNA from six representative clones was extracted from the small-scale cultures and digested with either EcoRI to release the entire PCR product or with SnaBI as a diagnostic indicator. Digestion with SnaBI should release a 306 bp fragment (SnaBI 476 to SnaBI 782) spanning the CMV promoter. The release of a second fragment mapping to the ITR junction (SnaBI 142 to SnaBI 476) is contingent on rearrangements that occur during formation of the concatomer, and could therefore range in size from 334 bp (2 complete ITR copies) to 0 bp if the ITRs have been deleted.

The PCR product from cell line 10-3.AV5 believed to contain 1.5 copies of AAV ITR (10-3.AV5) was also cloned into pCRII and digested with the indicated enzyme. This sample serves as a positive control for the diagnostic SnaBI digestion. Digestion of this sample with EcoRI correctly releases the 730 bp PCR fragment, as well as a secondary doublet band approximately 500 bp in size. This secondary band is believed to be an artifact due to secondary structure that develops in the 1.5 copies of AAV ITR during replication in bacteria. Digestion of the positive control with SnaBI releases the diagnostic 306 bp fragment from the CMV promoter and a 250 bp fragment that maps to the ITR junction.

Digestion of the six individual clones with EcoRI and SnaBI indicated deletions of variable lengths were present in all recovered junctions and largely confined to ITRs at the junctions. Sequence analyses further indicated that most deletions spanned portions of both ITRs at the junctions without involving contiguous viral DNA.

### E. Fluorescence in situ hybridization (FISH) Analysis

FISH was performed on cryosections of skeletal muscle to characterize the distribution of proviral DNA within the injected tissue. Small 4-5 mm pieces of muscle from treated or control mice were embedded in OTC and quickly frozen in liquid isopentane cooled with liquid nitrogen. Frozen sections, 10µ thick were cut on a cryomicrotome. Sections were mounted, fixed (Histochoice) and processed for fluorescence by *in situ* hybridization using a previously described protocol [E. Gussoni et al, Nat. Biotech., 14:1012-1016 (1996)]. Adjacent sections were stained for β-galactosidase activity to identify *lacZ* positive areas in muscle bundles.

For quantifying FISH signal, *lacZ* positive areas (as determined by staining adjacent sections for β-galactosidase activity) were examined under a Nikon microphot FxA microscope equipped with epifluorescence. The total number of individual muscle fibers corresponding to *lacZ* positive area in the section were counted under a standard phase contrast mode. The same area was then examined under fluorescence microscopy using the appropriate filter package for rhodamine isothiocynate. The number of muscle cell nuclei showing punctate staining was recorded. Each positive nucleus was examined under phase contrast to ascertain that it came from a muscle fiber. For controls, *lacZ* negative areas (areas in the same sections that lacked β-galactosidase activity, or mock transfected muscle sections) were examined and quantified in a similar manner.

For confocal microscopy, sections were observed under an oil immersion objective lens (100X) on a Leica confocal laser microscope equipped with Krypton-Argon laser (Leica lasertechnik, GmbH), TSC and Voxel View Silicon graphics central work stations. Images observed under rhodamine channel were also sequentially observed under differential interference contrast to confirm the location of fluorescence signal with muscle cell nuclei. The differential contrast and fluorescence images were then sequentially superimposed on a TCS central work station and were transferred to a Silicon graphics work station for image processing. Processed images were stored and printed using Photoshop software.

Serial sections were alternatively stained for β-galactosidase activity to identify transgene expressing muscle fibers and hybridized with a biotinylated proviral probe to localize the distribution of the proviral genome. A discrete fluorescent signal was detected in some nuclei of β-galactosidase expressing muscle fibers. A survey of three serial sections revealed hybridization in 53/1006 (5.3%) nuclei of β-galactosidase expressing fibers and 0/377 nuclei of fibers not expressing β-galactosidase. Hybridization was not detected in tissues from uninfected animals (data not shown).

The ability to detect viral genomes by FISH added another dimension to the analysis. Single foci of hybridization was detected in 5% of all nuclei contained within muscle fibers expression β-galactosidase. It is possible that this is an underestimate of transduced nuclei, because of limitations in sensitivity of this technique especially for target sequences less than 12 kb in size [B.J. Trask, Trends Genet., 7:149-154 (1991)]. The implications of the FISH analysis are interesting. The presence of 1 proviral genome/diploid myoblast genome, as measured by Southern, together with the FISH result that demonstrated 5% of nucleic acids harboring an AAV genome would predict that the average concatamer minimally comprises ten proviral genomes. These studies show β-galactosidase enzyme activity that extends far beyond the site of a vector transduced nucleus, suggesting an extended nuclear domain of at least 10 µm. This is consistent with previous work which documented extended nuclear domains for cytostolic proteins [H.M. Blau et al, Adv. Exp. Med. & Biol., 280:167-172 (1990]. An extended nuclear domain of transgene expression within the syncytial structure of the muscle fibers is important to applications of gene therapy for several reasons. The net yield of recombinant protein from a transduction event may be higher in a syncytium where the distribution of protein is less constrained by membrane barriers. Furthermore, this system has advantages in vector systems that require expression of multiple recombinant proteins such as those with inducible promoters [J.R. Howe et al, J. Biol. Chem., 270:14168-14174 (1995); V. M. Rivera et al, Nat. Med., 2:1028-1032 (1996)]. In muscle, coexpression of recombinant proteins does not require cotransduction with a single nucleus because of the extensive network of overlapping domains.

### Example 4 - Transgene Directed Immune Responses are Minimized when Helper-Free rAAV is Used for Muscle-Directed Gene Delivery

The stability of lacZ expression in muscle cells achieved from a lacZ-containing rAAV vector administered in the absence of helper virus was surprising in light of previous work which demonstrated destructive immune responses mounted against β-galactosidase expressed from adenoviral vectors in muscle fibers. Transgene-specific immune responses were studied further by measuring the serum levels of anti-β-galactosidase antibodies using Western analysis.

Blood was drawn from C57BL/6 and ROSA-26 mice (Jackson Laboratories, Bar Harbor, ME) necropsied on day 30 after virus injection and serum was collected. ROSA-26 is a transgenic line that carries the *E. coli* β-galactosidase cDNA and was developed on a 129 background. Serum was also harvested from C57BL/6 mice that received an intramuscular injection of either a recombinant LacZ adenovirus (H5.010CMVLacZ, 5 x 10⁸ pfu in 25 *µ*l of HBS) or the recombinant AAV.CMVLacZ (as described above). Both vectors express *E.coli β-*galactosidase from a CMV-driven minigene. Aliquots (5 *µ*g) of purified β-galactosidase from E. coli (Sigma) were resolved on a 10% SDS polyacrylamide gel (5 mg/lane) and electrophoretically transferred to a nitrocellulose membrane (Hybond-ECL, Amersham). The blot was incubated with blotto [5% nonfat milk, 50 mM Tris-HCl (pH 8.0), 2 mM CaCl, and 0.05% Tween-20] at room temperature for 2 hours to block available sites. Individual lanes were cut and incubated with serum (diluted 1:200 in blotto) for 1 hour at room temperature. Localization of antigen-antibody complex was accomplished by adding goat anti-mouse horseradish peroxidase conjugate, followed by ECL detection (Amersham). The cut lanes were reassembled on a mylar sheet prior to addition of ECL reagent and film documentation.

Intramuscular injection of the H5.010CMVlacZ E1 deleted adenovirus into skeletal muscle of C57BL/6 mice resulted in substantial β-galactosidase antibody accumulation in serum that did not occur in MHC-identical transgenic animals carrying an inserted lacZ gene which are immune tolerant to β-galactosidase. Significantly, neither C57BL/6 nor *lacZ* transgenic animals developed antibodies to β-galactosidase after intramuscular injection with AAV.CMVlacZ.

### Example 5 - Comparative Studies of Adenoviral and AAV Vectors in Muscle Cells

Studies of the biology of muscle-directed gene transfer mediated by recombinant AAV and adenovirus demonstrate that adenoviruses, but not AAV, infect antigen presenting cells (APCs), which elicit a cascade of immunological responses leading to destructive cellular and humoral immunity.

An experimental paradigm was constructed to define the specific differences in host responses to skeletal muscle-directed gene transfer with recombinant AAV and adenovirus. The goal was to delineate differences in the biology of these vector systems that lead to preferential immunologic activation directed against a transgene product (i.e., β-galactosidase) when expressed from a recombinant adenoviral vector, but not an AAV vector.

The general approach was to inject a *lac*Z expressing AAV into the right leg of a mouse. This has been shown in the examples above to confer efficient and stable gene expression. In other experimental groups, the animals receive rAAV in addition to various combinations of vectors and cells in order to define components of the immune response directed against Ad that lead to destructive cellular and humoral immunity. The effects of these experimental manipulations were followed by assessing their impact on the stability of the rAAV engrafted muscle fibers, as well as measuring other immunologic parameters. Any intervention that elicits immunity to β-gal in muscle fibers can be detected by assessing the stability of transgene expression in the AAV-transduced muscle, and the development of inflammation.

Four experimental groups were developed for this study as summarized below. Virus was injected into mice using techniques substantially similar to those described in Example 2 above, i.e., virus was suspended in phosphate-buffered saline and injected directly into the tibialis anterior muscles. When the animals were necropsied, muscle tissues were snap-frozen in liquid nitrogen-cooled isopentane and sectioned at 6 µm thickness, while serum samples and the draining inguinal lymph nodes were harvested for immunological assays.

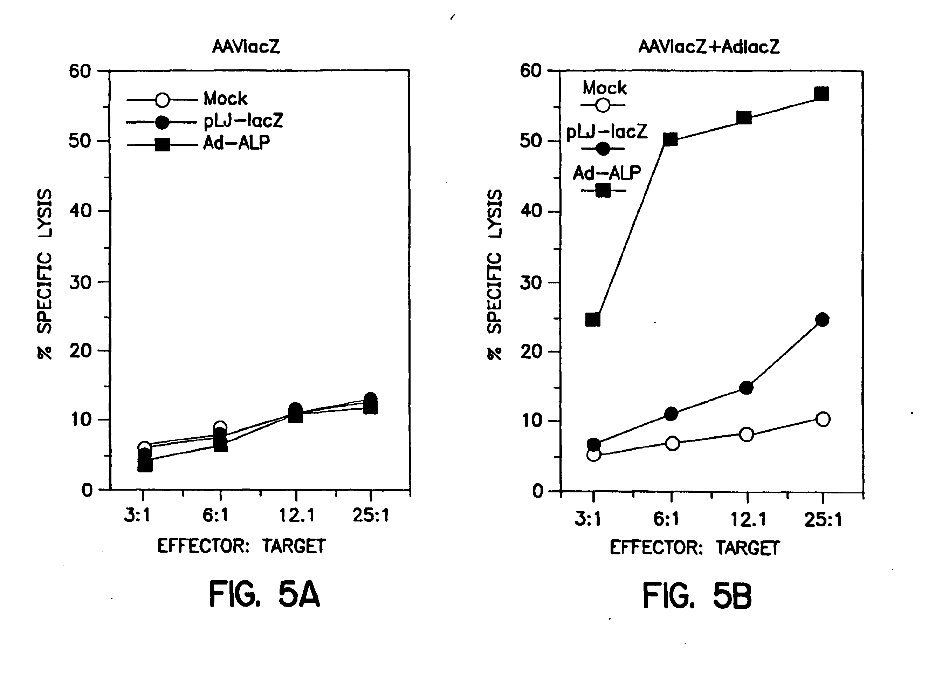

Lymphocytes were harvested from inguinal lymph nodes and a standard 6 hr ⁵¹chromium (Cr)-release assay was performed essentially as described below, using different ratios of effector to target cells (C57SV, H-2^{b}) in 200 *µ*l DMEM in V-bottom 96-well plates. Prior to mixing with the effector cells, target cells were either infected with an adenovirus expressing alkaline phosphatase (Ad*ALP*) or stably transduced with a lacZ-expressing retrovirus, pLJ-lacZ, labeled with 100 µCi of ⁵¹Cr and used at 5 x 10³ cells/well. After incubation for 6 hr, aliquots of 100 *µ*l supernatant were counted in a gamma counter. The results for groups 1-3 are provided in Figs. 5A-5C.

Frozen sections (6µm) were fixed in methanol and stained with anti-CD4 and anti-CD8 antibodies. Morphometric analysis was performed to quantify the number of CD8+ cell and CD4+ cells per section.

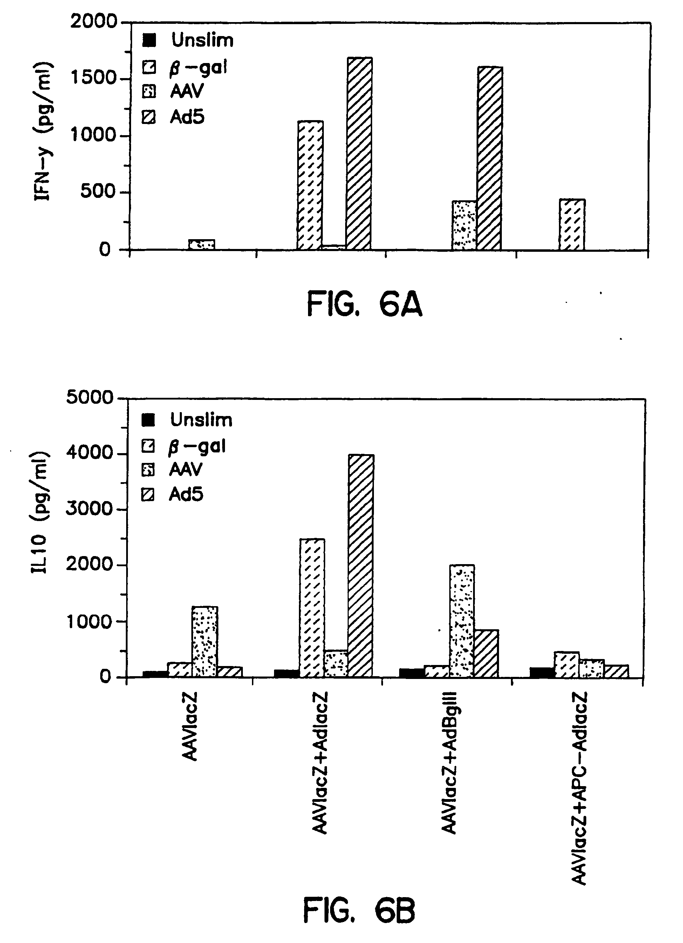

The cytokine release assay was performed essentially as follows. Lymphocytes were restimulated for 40 hr with β-galactosidase, purified AAV, or adenovirus type 5. Cell-free supernatants (100 µl) were assayed for the secretion of IL-10 and IFN-γ. Proliferation was measured 72 hr later by a 8 hr ³H-thymidine (0.50 µCi/well) pulse. The results for the four groups are provided in Figs. 6A and 68.

The neutralizing antibody assay was performed essentially as follows. Mouse serum samples were incubated at 56°C for 30 min to inactivate complement and then diluted in DMEM in two-fold steps starting from 1:20. Each serum dilution (100 *µ*l) was mixed with **β-**galactosidase or adenovirus type 5. After 60 minincubation at 37°C, 100 *µ*l of DMEM containing 20% FBS was added to each well. Cells were fixed and stained for β-galactosidase expression in the following day. All of the cells stained blue in the absence of serum samples. The results for the four groups are provided in Figs. 6A and 6B.

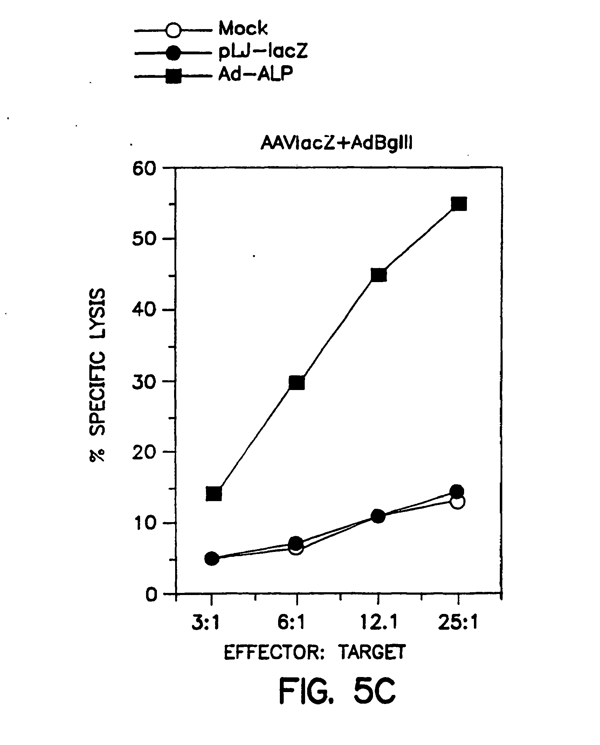

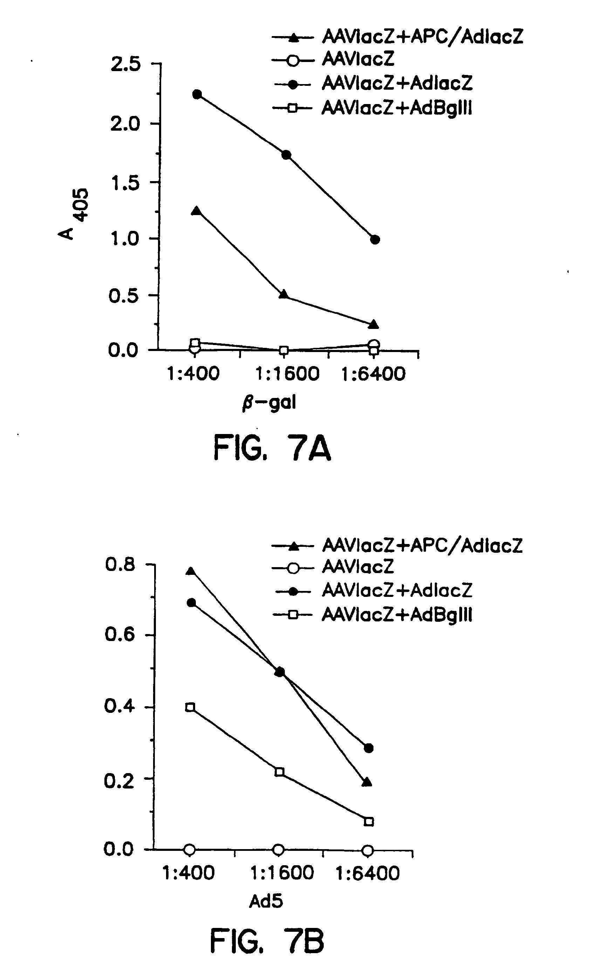

Group 1 mice received AAV.CMV*lacZ*, produced as described in Example 1, in the right leg with no other intervention. Transduction with AAV.CMV*lacZ* alone led to high levels of stable gene transfer (evident even at 28 days) without infiltration of lymphocytes. No activation of CD8 T cells was detected (Fig. 5). Nor were antigen-specific, CD4⁺ T cells [i.e., viral or β-galactosidase (Figs. 6A and 6B)] detected. Antibodies were not generated to β-galactosidase or adenovirus (Figs. 7A and 7B).

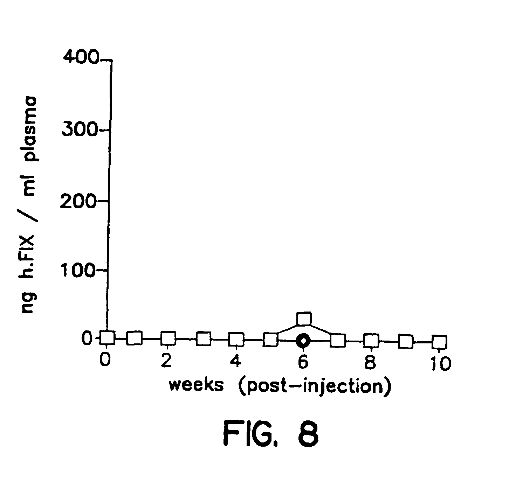

Group 2 mice received AAV.CMV*lacZ* in the right leg and adenovirus expressing *lacZ* (H5.010CMVlacZ) in the left leg. The goal of this group was to determine if the immunologic response to the Ad-infected muscle fibers was systemic, as demonstrated by its impact on the biology of the contralateral AAV.CMV*lacZ* transduced leg. Apparently, the adenoviral *lacZ* treatment induced an immune response to β-galactosidase that led to the destruction of the AAV *lacZ* transduced fibers. Not surprisingly, this was associated with infiltration of both CD4 and CD8 T cells into the AAV transduced leg, and the activation of cytotoxic T lymphocytes to both adenoviral and β-galactosidase antigens (Fig. 8). Activated CD4 T cells specific for AAV, Ad, and β-gal antigens and antibodies specific for adenovirus and β-galactosidase were also observed.

Group 3 animals received a mixture of AAV.CMV*lacZ* and Ad BglII in the right leg. AdBgIII is an E1-deleted adenovirus that expresses no recombinant gene. The goal of this group was to determine if the adenovirus provides an adjuvant effect which would elicit immunity to AAV to *lacZ* in this setting. This did not lead to loss of transgene expression, although there was substantial infiltration of CD8 T cells and some activation of CD4 T cells to viral antigens, but not to β-galactosidase (Figs. 6A-6B). As expected, antibodies were generated towards adenovirus but not towards β-galactosidase (Figs. 7A-7B).

Group 4 animals received AAV.CMV*lacZ* in the right leg and were adoptively transferred with antigen presenting cells harvested from naive animals and infected *ex vivo* with adenovirus.

These animals mounted a vigorous and effective immunologic response to β-gal, as demonstrated by the loss of transgene expression, and the massive infiltration of CD8 and CD4 T cells. CD4 T cells were activated to β-gal in this experiment as shown in Figs. 6A-6B, and anti-β-galactosidase antibodies generated, as shown in Figs. 7A-7B.

### Example 6 - Transduction of a purified rAAV vector containing Factor IX into skeletal muscle cells and expression of F.IX at therapeutically useful levels and without eliciting a cytotoxic immune response.

The data provided in this example demonstrates that the method of this invention provides prolonged expression of a therapeutic transgene, F.IX, in both immunocompetent and immunoincompetent subjects in the absence of an immune response cytotoxic to the transduced cells. Further, the protein levels achieved in the serum of immunoincompetent animals are adequate to achieve a therapeutic effect. Thus, prolonged expression of human F.IX in muscle cells via rAAV vectors in immunoincompetent patients, such as those with hemophilia B, is useful to deliver F.IX to patients with that disease.

### A. Preparation of purified rAAV

The rAAV vector used in the following *in vivo* experiments carries an expression cassette containing the human F.IX cDNA including a portion of Intron I under transcriptional control of the cytomegalovirus (CMV) immediate early gene promoter/enhancer and SV40 transcription termination signal. The vector, which contains this expression cassette flanked by AAV ITR sequences, and which completely lacks AAV protein coding sequences, was constructed as follows.

Recombinant AAV was generated by co-transfection of a F.IX *cis* plasmid (pAAV-FIX) and the trans-acting plasmid pAAV/Ad [A.W. Skulimowski and R.J. Samulski, Method. Mol. Genet., 7:7-12 (1995)] into human embryonic kidney (293) cells infected with an E1-deleted adenovirus as described by Fisher et al., J. Virol., 70:520-532 (1996). pAAV-FIX was derived from psub201 [Skulimowski and Samulski cited above] and contains the CMV promoter/enhancer, the human F.IX coding sequence including 1.4-kb fragment of Intron I [S. Kurachi et al, J. Biol. Chem., 270:5276-5281 (1995)], and the SV40 polyadenylation signal, flanked by AAV ITR sequences. The AAV rep and cap gene functions were supplied in *trans* by pAAV/Ad. The E1-deleted adenovirus contained a β-galactosidase (LacZ) or alkaline phosphatase (ALP) reporter gene to trace potential contamination of rAAV stocks with this helper virus. Cells were lysed 48 hours after transfection by sonication, and the released rAAV particles were purified by four rounds of CsCl density gradient centrifugation as described by Fisher et al., cited above.

The resulting rAAV-F.IX particles had a density of 1.37-1.40 g/ml. The titer of the purified rAAV-F.IX was determined by slot blot hybridization using a probe specific to either the CMV promoter or Intron I sequences and standards of pAAV-F.IX plasmid DNA of known concentration. The ability of rAAV-F.IX to transduce cells *in vitro* was confirmed by transducing growing HeLa cells and measuring the concentration of hF.IX in the culture supernatant 36 hours post-infection with an ELISA specific to hF.IX [J. Walter et al, Proc. Natl. Acad. Sci. USA, 93:3056-3061 (1996)]. rAAV-F.IX (10¹²-10¹³ genomes/ml) was stored at -79°C in HEPES-Buffered Saline, pH 7.8, including 5% glycerol.

Purified rAAV-F.IX routinely lacked detectable amounts of contaminating adenovirus when analyzed by transduction of 293 cells followed by staining for alkaline phosphatase or β-galactosidase as described by Fisher et al., cited above. Wild-type AAV was detected at <1 infectious unit per 10⁹ genomes of rAAV-F.IX. The assay for wild-type AAV was as follows: 293 cells grown on chamber slides were co-infected with adenovirus and with aliquots of purified rAAV-F.IX and fixed for immunofluorescence staining 24 hours post-infection. A mouse monoclonal antibody against AAV capsid proteins (American Research Products, Belmont, MA) served as a primary antibody, and anti-mouse IgG (DAKO Corporation, Carpinteria, CA) in a dilution of 1:40 as secondary antibody.

### B. Introduction of rAAV into skeletal muscle

Mouse strains selected for intramuscular injection with rAAV were C57BL/6 (Charles River Laboratories, Wilmington, MA) and B6, 129, Rag 1 (Jackson Laboratories, Bar Harbor, Maine). Female mice (4-6 week-old) were anesthetized with an intraperitoneal injection of ketamine (70 mg/kg) and xylazine (10 mg/kg), and a 1 cm longitudinal incision was made in the lower extremity. AAV-F.IX (2x10¹¹ or 1x10¹⁰ vector genomes/animal in HEPES-buffered saline, pH 7.8) was injected into the tibialis anterior (25 *µ*l) and the quadriceps muscle (50*µ*l) of each leg using a Hamilton syringe. Incisions were closed with 4-0 Vicryl suture. Blood samples were collected at seven-day intervals from the retro-orbital plexus in microhematocrit capillary tubes and plasma assayed for hF.IX by ELISA (part C below). For immunofluorescence staining (part D below) and DNA analysis (part F below), animals were sacrificed at selected time points and injected and non-injected muscle tissue was excised. Tissue was placed in OTC embedding compound, snap-frozen in liquid nitrogen-cooled isopentane for seven seconds, and immediately transferred to liquid nitrogen.

### C. Detection of human F.IX by ELISA

Human F.IX antigen in mouse plasma was determined by ELISA, as described by Walter et al., cited above. This ELISA did not cross-react with mouse F.IX. All samples were measured in duplicate. Protein extracts from injected mouse muscle were prepared by maceration of muscle in phosphate buffered saline (PBS) containing leupeptin (0.5 mg/ml) followed by sonication. Cell debris was removed by microcentrifugation, and 1:10 dilutions of the protein extracts were assayed for hF.IX by ELISA. Extracts from rAV.CMVLacZ (see Example 1 above)-injected muscle were used as negative controls. Protein concentrations were determined with the BIORAD assay (Bio-Rad, Hercules, CA).

### D. Immunofluorescence staining

In order to perform immunofluorescence staining of tissue sections, cryosections of muscle tissue (6µm) were fixed for 15 minutes in 3% paraformaldehyde in PBS, pH 7.4, rinsed in PBS for 5 minutes, incubated in methanol for 10 minutes, washed three times in PBS, and then blocked in PBS/3% bovine serum albumin (BSA) for 1 hour. Sections were subsequently incubated overnight with an affinity purified goat anti-human F.IX antibody (Affinity Biologicals) that was diluted 1:1000 in PBS/1% BSA. After three washes (10 minutes each) in PBS/1% BSA, the secondary antibody was applied for 90 minutes (FITC-conjugated rabbit anti-goat IgG, DAKO Corporation, diluted 1:200 in PBS/1% BSA). After three additional washes in PBS/1% BSA, sections were rinsed in distilled water, air-dried and mounted with Fluoromount G mounting media (Fisher Scientific). All incubation steps were at room temperature, except for incubation with the primary antibody (4°C). The same protocol was applied when sections were stained with rabbit anti-human collagen IV as primary antibody (Chemicon, Temecula, CA) in a 1:500 dilution and FITC conjugated anti-rabbit IgG (DAKO Corporation) as secondary antibody. For co-localization studies, a goat anti-hF.IX antibody conjugated to FITC (Affinity Biologicals) was applied simultaneously with the anti-collagen IV antibody, and rhodamine-conjugate anti-rabbit IgG (Chemicon) was used to detect collagen IV-antibody complexes. Fluorescence microscopy was performed with a Nikon FXA microscope.

### E. Tests for circulating antibody against hF.IX

Plasma samples of C57BL/6 mice intramuscularly injected with AAV-F.IX were tested for the presence of antibodies against hF.IX using the ELISA. Microtiter plates were coated with human F.IX (1 µg/ml in 0.1M NaHCO₃, pH 9.2). Dilute plasma samples (1:16) were applied in duplicate, and antibodies against hF.IX detected with horseradish peroxidase conjugated anti-mouse IgG (Zymed, San Francisco, CA) in a dilution of 1:2000. Buffer conditions were as described in Walter et al, cited above. Anti-hF.IX levels were estimated by comparison of absorbance values with monoclonal mouse anti-hF.IX (Boehringer Mannheim) diluted to a final concentration of 1 µg/ml. Western blots to demonstrate the presence of anti-hF.IX were performed as outlined by Dai et al., Proc. Natl. Acad. Sci. USA, 92:1401-1405 (1995), except that a horseradish peroxidase conjugated goat anti-mouse IgG antibody (Boehringer Mannheim) was used as secondary antibody, thereby allowing the detection of hF.IX-antibody complexes with ECL reagent (Amersham). Dilution of mouse plasma were 1:500.

### F. DNA analyses

Genomic DNA was isolated from injected muscle tissue as described for mammalian tissue by Sambrook et al., Molecular Cloning: A Laboratory Manual, Cold Spring Harbor Press, Cold Spring Harbor, NY (1989). As described in Example 3 of the application, PCR reactions were carried out in order to amplify head-to-tail junctions of rAAV tandem repeats. The forward primer 005 [SEQ ID NO:4] anneals the SV40 polyadenylation signal (bp position 8014-8033), and reverse primers 013 [SEQ ID NO: 2] and 017 [SEQ ID NO: 3] bind to the CMV promoter (bp position 4625-4606 and 4828-4809). PCR reactions were performed using 100 ng genomic DNA in a total reaction volume of 100 µl including 1.5 mM MgCl₂, and 0.5 µM of primer pair 005/013 or 005/017. After an initial denaturation step (94°C for four minutes), 35 cycles of the following profile were carried out: denaturation at 94°C for four minutes), 35 cycles of the following profile were carried out: denaturation at 94°C for 1 minutes, annealing at 52°C for 1 minute, extension at 72°C for 90 seconds (10 minutes during the final cycle). PCR products were cloned (for DNA sequence analysis) using the T/A cloning kit (Invitrogen, San Diego, CA). Southern blot hybridizations were performed using ³²P-dCTP random primed labelled probes specific for the CMV promoter (for hybridization to PCR fragments) or for intron I of hF.IX as present in rAAV-F.IX (for hybridization to genomic mouse DNA).

### G. Results of expression of hF.IX in immunocompetent mice

Immunocompetent C57BL/6 mice were injected intramuscularly with rAAV-hF.IX and the animals sacrificed one month post-injection. An ELISA on protein extracts from injected muscles (tibialis anterior and quadriceps) demonstrated the presence of 1.8-2.1 ng hF.IX/mg tissue (40-50 ng hF.IX/mg protein). The expression of hF.IX in muscle tissues was confirmed by immunofluorescence studies on tissue sections.

Immunocompetent C57BL/6 mice were injected intramuscularly with rAAV-hF.IX and the animals sacrificed three months post-injection. Factor IX was not detected in uninjected muscle. Factor IX was not detected in muscle injected with a control, rAAV-lacZ. Expression of human F.IX was detected in muscle fibers of C57BL/6 mice three months post-injection (3.3x10¹⁰ vector genomes per injection site; magnification 200x). Note that F.IX is present not only in the muscle fibers themselves, but in the interstitial spaces between the fibers as well where it appears to accumulate.

Interestingly, this staining pattern was identical to that seen with a polyclonal antibody against human collagen IV, which also stained the interstitial space. A stain of muscle one month post infection with rAAV-hF.IX (3.3 x 10¹⁰ genomes per injection site) stained well with antibody to human collagen IV (data not shown). Collagen IV has recently been identified as a binding protein for human F.IX [W.-F. Cheung et al, Proc. Natl. Acad. Sci, USA, 93:11068-11073 (1996)].

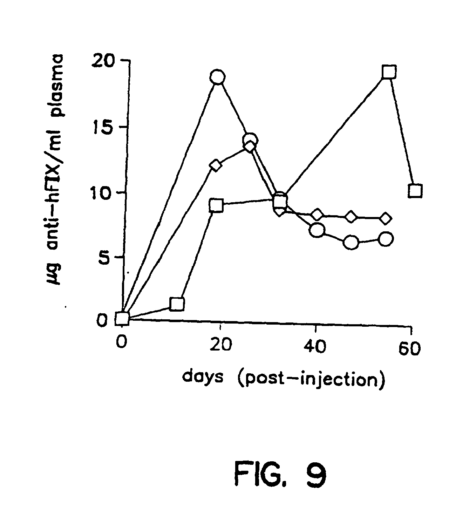

Initial experiments in immunocompetent mice demonstrated that, despite high levels of gene transfer and stable expression of hF.IX in injected muscle, it was not possible to detect significant amounts of hF.IX in the circulation by ELISA. See, Fig. 8. Further experiments demonstrated that the animals had developed high-titer antibodies against the circulating foreign protein. For example, when the same plasma samples were tested for antibodies against human F.IX, a strong antibody response was seen in all injected animals starting at day 11 post-injection. See Fig. 9. Using Western blot analysis, high levels of circulating antibody were found to have persisted for the duration of the experiment.

This finding contrasted with our previously reported experience, in which a different route of administration, i.e., intravenous injection, of a different vector, i.e., an adenoviral vector expressing hF.IX, effected a different immune response, i.e., did not trigger formation of neutralizing antibodies against hF.IX (Walter et al, cited above)].

Levels of protein expression required to induce antibody formation however are quite low. The Western blot assay is somewhat less sensitive than an ELISA but documents what appears to be a rising antibody titer beginning 18 days after injection. Thus in the immunocompetent animals, the absence of detectable hF.IX. expression at initial time points is a result of the biology of rAAV expression in muscle, whereas the subsequent absence of detectable F.IX in the circulation results from the production of antibodies to the foreign protein. Nevertheless the serum antibody response was not associated with cytotoxic immune response directed against the transgene-expressing cells. In fact, neither inflammation nor extensive tissue damage was observed in any of the tissue sections discussed above nor in sections analyzed by H&E staining (data not shown). This contrasts with the immune response elicited by injection of skeletal muscle with recombinant adenovirus carrying a transgene [Dai et al, cited above; Y. Yang et al, Hum. Molec. Genet., 5:1703-1712 (1996); X. Xiao et al, J. Virol., 70:8098-8108 (1996)].

### H. The expression of hF.IX in immunodeficient mice

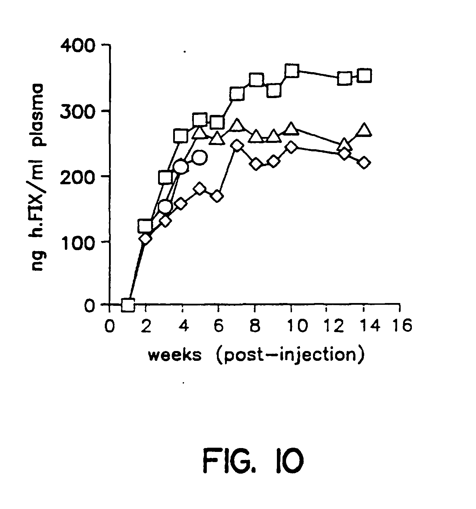

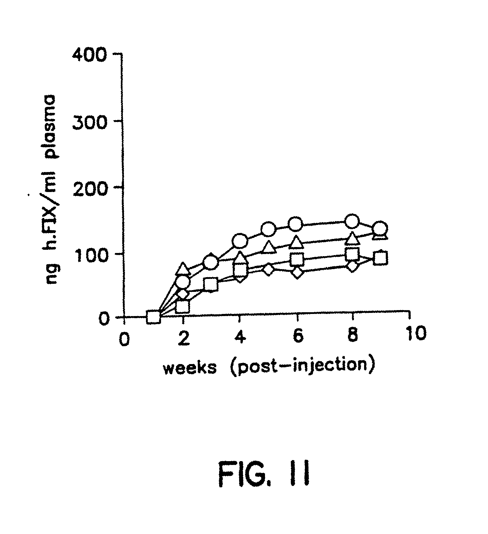

AAV-F.IX was delivered to muscles of Rag 1 mice, which are homozygous for a mutation in the recombinase activating gene 1. These animals are therefore functionally equivalent to severe combined immunodeficiency (SCID) mice and do not produce mature B or T cells. A dose of 2x10¹¹ rAAV-hF.IX vector genomes per animal resulted in stable expression of hF.IX in mouse plasma. See Fig. 10. Human F.IX was first detectable by ELISA in the second week after the injection and rose gradually thereafter. Plasma levels in all animals reached a plateau of therapeutic levels of F.IX five to seven weeks post-injection at 200 to 350 ng hF.IX/ml mouse plasma. This level was maintained for the duration of the experiment (four months post-injection). When a total of 1x10¹⁰ rAAV-hF.IX vector genomes was injected, expression was three- to four-fold lower, but still reached therapeutic levels (>100 ng/ml) for some animals. See, Fig. 11. These levels, which represent 4-7% of normal circulating levels in plasma, are well within a therapeutic range, and demonstrate that the method of this invention is a feasible for the treatment of hemophilia, a disease associated with immunodeficiency.

### I. Results of DNA Analysis

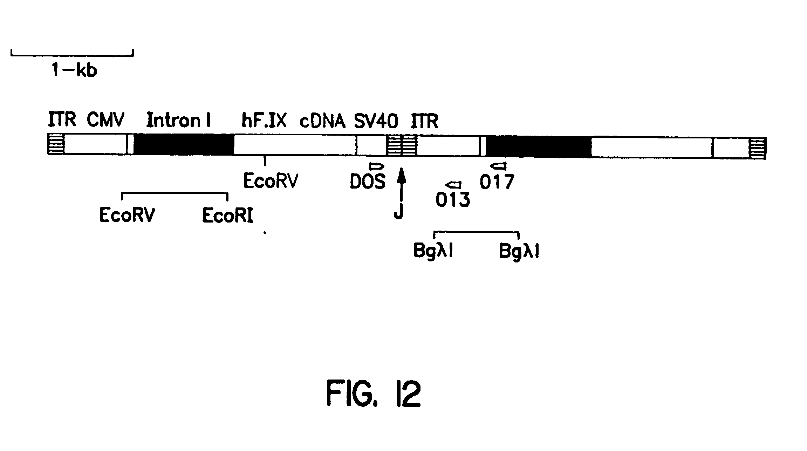

Genomic DNA from injected muscle tissue was isolated six to eight weeks post-injection. The presence of introduced vector DNA was demonstrated by digestion with EcoRV, which releases a 1.8-kb fragment from the vector construct including the entire 1.4-kb intron I sequence. A probe specific to intron I hybridized to this fragment and did not cross-hybridize to mouse DNA from an uninjected animal. Undigested DNA showed as hybridization signal in the high molecular weight DNA. Furthermore, PCR primers designed to amplify junction sequences of head-to-tail concatamers of recombinant AAV present in transduced cells (Fig. 12) successfully amplified such sequences from muscle DNA isolated from AAV-F.IX transduced tissue (tibialis anterior and quadriceps of immunodeficient and immunocompetent animals). The PCR products were visualized by Southern blot hybridization with a probe specific to the CMV promoter/enhancer. Primer pair 005-013 produced fragments that were 1.0-kb and smaller; primer pair 005-017 amplified fragments that were 1.2-kb and smaller. As expected, these PCR reactions resulted not in distinct bands of the sizes noted above, but rather in a series of amplification products with a maximum size as predicted, because of imprecise joining of AAV genomes present in these tandem repeats [S. K. McLaughlin et al, J. Virol., 62:1963-1973 (1988)]. The imprecise joining results from variable deletions of ITR sequences at the junction sites as confirmed by DNA sequencing of cloned PCR products (data not shown).

### J. PCR studies

While head-to-head and tail-to-tail arrangements of AAV genomes can occur during viral replication [K. I. Berns, Microbiol. Rev., 54:316-329 (1990)], head-to-tail arrays are more typically associated with AAV that has been integrated into the chromosomal DNA of the transduced cell during latent infection [S. K. McLaughlin et al, J. Virol., 62:1963-1973 (1988); J. D. Tratschin et al, Mol. Cell Biol., 5:3251-3260 (1985); N. Muzcyka, Current Topics in Microbiology and Immunology, 158:97-129 (1992)].

Southern blot data on undigested rAAV-injected muscle cell DNA demonstrated that the rAAV DNA derived from host cell genomic DNA six weeks after injection is present as a high molecular weight species. The higher signal intensity seen with the restricted DNA likely results from an unmasking effect when the fragments are separated from the bulk of genomic DNA [X. Xiao et al, J. Virol., 70:8089-8108 (1996)]. This finding, the presence of a hybridization signal for high molecular weight DNA, is, like the PCR data, consistent with integrative events occurring during transduction. The integration state of the rAAV discussed in this paragraph and in paragraph I is also likely to contribute to the stability and prolonged expression of the transgene.

### Example 7 - Expression of ApoE using rAAV Administered to a Skeletal Muscle Cell

The following example demonstrates the prolonged expression of another therapeutic transgene product, apolipoprotein E (ApoE), a protein useful in the treatment of atherosclerosis, by introduction into skeletal muscle of a rAAV vector according to this invention. Again, the absence of a destructive CTL response permits the prolonged expression of the transgene product.

### A. Construction of the rAAV

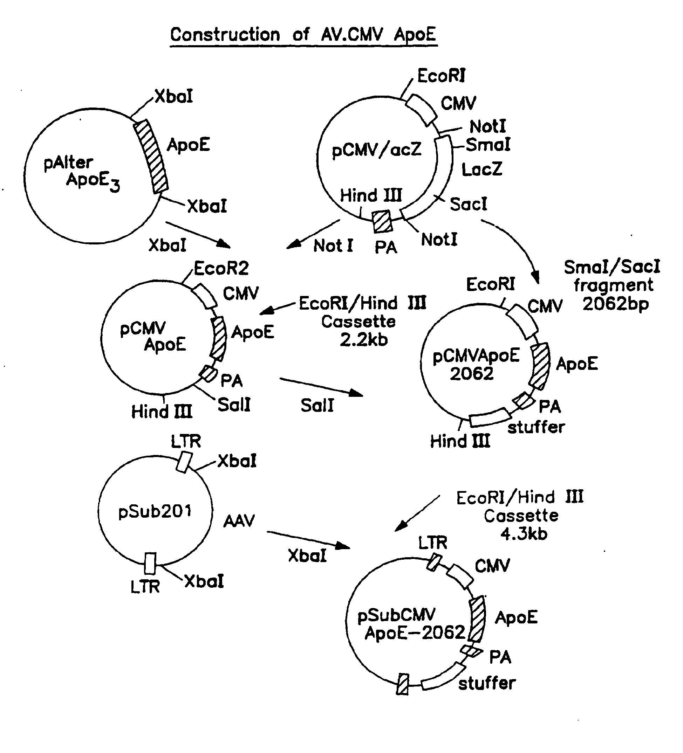

Recombinant AAV vectors encoding the secreted protein human ApoE were constructed in a manner similar to that described above for F.IX. ApoE cDNA was excised from a plasmid pAlterApoE3 (contributed by Dr. Rader's laboratory, University of Pennsylvania) with XbaI digestion, blunted and cloned into NotI digested pCMVLacZ backbone (see the vector construction diagram of Fig. 13). A 2062 bp SmaI/SacI fragment from the lacZ gene in pCMVLacZ was isolated and inserted into the SalI site of the new plasmid as a stuffer. The ApoE minigene cassette, which now contained the CMV promoter, ApoE cDNA SV40 polyadenylation sequences and a 2062 bp stuffer, was isolated by EcoRI/HindIII digestion (total length: 4.3 kb) and then ligated to an XbaI digested pSub201 backbone. The final product, designated pSubCMVApoE-2062RO, was used as the cis plasmid in the production of rAAV.ApoE production following the procedures described above for rAAV.F.IX.

### B. In vitro analysis-of ApoE expression by rAAV.AnoE

84-31 cells seeded in 6 well plates were infected with 2 microliters of CsCl purified rAAV.ApoE in 2 ml Dulbeccos Modified Eagles Medium with 2% fetal bovine serum. The cells were kept at 37°C for 48 hours. An aliquot of the supernatant was then removed from the well for a Western blot analysis of ApoE. The results showed ApoE protein was clearly detectable in the supernatant of 84-31 cells infected with AAV.ApoE.

### C. In vivo expression of rAAV.ApoE in ApoE knockout mice

2.5 to 5 X 10¹⁰ particles of rAAV.ApoE were injected into anterior tibialis and quadriceps muscles on each side of ApoE knockout mice (total particles per mouse: 5 X 10¹⁰ to 5 x 10¹¹) . Prolonged local ApoE expression was detectable by immunofluorescence at day 28 and day 120 post-injection even in the presence of plasma anti-ApoE antibodies.

### D. Conclusion

This experiment demonstrates that intramuscular injection of the vector, rAAV-ApoE, into Apo-E knockout mice leads to muscle fiber transduction and secretion of substantial quantities of the recombinant protein in the circulation. Prolonged expression of the transgene in the muscle fiber in the absence of a destructive CTL immune response was achieved, even though humoral immunity was elicited to the secreted protein.

### Example 8 - Transgene expression in a primate

A rhesus monkey was anesthetized, the forearm was clipped and aseptically prepared and a 0.5 cm incision was made in the skin over the tibialis anterior muscle. The fascia was identified and the rAV.CMVLacZ (described in Example 1) viral suspension (175 microliter of 10² genomes/ml) was injected 5-7 mm deep into the fascia. Fourteen days later, a muscle biopsy was removed, frozen in OCT, sectioned and stained in X-gal. Tissue sections were quantitatively analyzed using the Leica Z500MC Image Processing and Analysis System interfaced with a Nikon FXA Microscope. X-gal histochemistry revealed high level β-galactosidase expression in the majority of muscle fibers in the area of the injection site. Twenty percent of fibers expressed β-galactosidase in a 224 mm² area in the region of the injections. Based on the above-described results with ApoE and F.IX, expression is expected to be prolonged in the absence of a cytotoxic immune response. These data support that the expression of a transgene according to this invention can be duplicated in an animal other than a mouse, and particularly in a primate animal.

## Claims

1. Use of a recombinant adeno-associated virus (rAAV) comprising a heterologous nucleic acid sequence encoding a product operably linked to sequences which control expression thereof in a cell for the manufacture of a medicament for the treatment of a chronic disorder, wherein the medicament is in a form adapted for delivery of the rAAV to a muscle cell which expresses the product and the rAAV is purified from contaminating helper adenovirus so that the product is expressed in the absence of a destructive immune response to the product, and wherein the medicament is adapted for delivery by repeated administration, and the product is a neoantigen.

2. Use of a recombinant adeno-associated virus (rAAV) according to claim 1, wherein the rAAV is as free of contamination with helper adenovirus as can be achieved by four rounds of cesium chloride gradient centrifugation.

3. Use according to claim 1 or 2, wherein the heterologous nucleic acid encodes a therapeutic product and the medicament provides prolonged expression of the product.

4. Use according to any one of the preceding claims, wherein the heterologous nucleic acid sequence encodes a secretable protein.

5. Use according to claim 4, wherein the secretable protein is selected from factor IX, apolipoprotein E, β-interferon, insulin, erythropoietin, growth hormone and parathyroid hormone.

6. Use according to any one of the preceding claims, wherein the rAAV further comprises AAV 5' and 3' inverted terminal repeats flanking the heterologous nucleic acid sequence.

7. Use according to any one of the preceding claims, wherein the rAAV comprises, from 5' to 3', 5' AAV inverted terminal repeats (ITRs), a heterologous promoter, the heterologous nucleic acid sequence, a polyadenylation sequence, and 3' AAV ITRs.

8. Use according to claim 7, wherein the heterologous promoter is a constitutive promoter.

9. Use according to claim 8, wherein the constitutive promoter is selected from the cytomegalovirus immediate early promoter and the Rous sarcoma LTR promoter.

10. Use according to claim 7, wherein the heterologous promoter is an inducible promoter.

11. Use according to any one of the preceding claims, wherein the muscle is selected from skeletal muscle, cardiac muscle and smooth muscle.

12. Use according to any one of the preceding claims, wherein the medicament comprises at least 10⁹ particles of rAAV.

13. Use according to any one of the preceding claims, wherein the medicament comprises 2.5 x 10¹⁰ to 5 x 10¹⁰ particles of rAAV.

14. Use according to claim 13, wherein the medicament is injected into the muscle cells at a dose of 2 x 10¹¹ vector genomes of rAAV.

15. Use according to any one of claims 1 to 6, wherein the chronic disorder is hemophilia and the product is factor IX.

16. Use according to any one of claims 1 to 6, wherein the chronic disorder is atherosclerosis and the product is apolipoprotein E.

## Patentansprüche

1. Verwendung eines rekombinanten Adeno-assoziierten Virus (rAAV), umfassend eine heterologe Nukleinsäuresequenz, die für ein Produkt codiert, in funktionsfähiger Verbindung mit Sequenzen, die die Expression hiervon in einer Zelle kontrollieren, für die Herstellung eines Medikaments zur Behandlung einer chronischen Erkrankung, wobei das Medikament in einer Form vorliegt, die angepasst ist für die Auslieferung des rAAV an eine Muskelzelle, die das Produkt exprimiert, und wobei das rAAV von kontaminierendem Helfer-Adenovirus gereinigt wird, sodass das Produkt in Abwesenheit einer schädlichen Immunantwort gegen das Produkt exprimiert wird, und wobei das Medikament angepasst ist für eine Zuführung durch wiederholte Verabreichung, und wobei das Produkt ein Neoantigen ist.

2. Verwendung eines rekombinanten Adeno-assoziierten Virus (rAAV) nach Anspruch 1, wobei das rAAV so frei von der Kontamination mit Helfer-Adenovirus ist, wie dies durch vier Runden einer Caesiumchloridgradienten-Zentrifugation erreicht werden kann.

3. Verwendung nach Anspruch 1 oder 2, wobei die heterologe Nukleinsäure ein therapeutisches Produkt codiert und das Medikament eine verlängerte Expression des Produkts bereitstellt.

4. Verwendung nach einem der vorigen Ansprüche, wobei die heterologe Nukleinsäuresequenz für ein sekretierbares Protein codiert.

5. Verwendung nach Anspruch 4, wobei das sekretierbare Protein ausgewählt ist aus Faktor IX, Apolipoprotein E, β-Interferon, Insulin, Erythropoietin, Wachstumshormon und Nebenschilddrüsenhormon.

6. Verwendung nach einem der vorigen Ansprüche, wobei das rAAV weiterhin AAV 5' und 3' invertierte terminale Sequenzwiederholungen umfasst, die die heterologe Nukleinsäuresequenz flankieren.

7. Verwendung nach einem der vorigen Ansprüche, wobei das rAAV, von 5' nach 3', 5' AAV invertierte terminale Sequenzwiederholungen (ITRs), einen heterologen Promotor, die heterologe Nukleinsäuresequenz, eine Polyadenylierungssequenz und 3' AAV ITRs umfasst.

8. Verwendung nach Anspruch 7, wobei der heterologe Promotor ein konstitutiver Promotor ist.

9. Verwendung nach Anspruch 8, wobei der konstitutive Promotor ausgewählt ist aus dem sehr frühen ("immediate early") Promotor des Cytomegalievirus und dem Rous-Sarkoma LTR-Promotor.

10. Verwendung nach Anspruch 7, wobei der heterologe Promotor ein induzierbarer Promotor ist.

11. Verwendung nach einem der vorigen Ansprüche, wobei der Muskel ausgewählt ist aus Skelettmuskel, Herzmuskel und glattem Muskel.

12. Verwendung nach einem der vorigen Ansprüche, wobei das Medikament wenigstens 10⁹ Partikel von rAAV umfasst.

13. Verwendung nach einem der vorigen Ansprüche, wobei das Medikament 2,5 x 10¹⁰ bis 5 x 10¹⁰ Partikel von rAAV umfasst.

14. Verwendung nach Anspruch 13, wobei das Medikament in einer Dosis von 2 x 10¹¹ Vektorgenomen von rAAV in die Muskelzellen injiziert wird.

15. Verwendung nach einem der Ansprüche 1 bis 6, wobei die chronische Erkrankung Hämophilie ist und das Produkt Faktor IX ist.

16. Verwendung nach einem der Ansprüche 1 bis 6, wobei die chronische Erkrankung Arteriosklerose ist und das Produkt Apolipoprotein E ist.

## Revendications

1. Utilisation d'un virus adéno-associé recombinant (rAAV) comprenant une séquence d'acide nucléique hétérologue codant un produit fonctionnellement lié à des séquences qui contrôle l'expression de celui-ci dans une cellule, pour la fabrication d'un médicament destiné au traitement d'une maladie chronique, le médicament se présentant sous une forme adaptée à la délivrance du rAAV en direction d'une cellule musculaire exprimant le produit et le rAAV étant purifié de toute contamination par un adénovirus auxiliaire, de façon à ce que le produit s'exprime en l'absence de toute réponse immunitaire destructrice au produit, le médicament étant, en outre, adapté à une délivrance impliquant une administration répétée et le produit étant un néoantigène.

2. Utilisation d'un virus adéno-associé recombinant (rAAV) selon la revendication 1, dans laquelle le rAAV est aussi purifié de toute contamination par un adénovirus auxiliaire que possible suite à quatre cycles de centrifugation sur un gradient de chlorure de césium.

3. Utilisation selon la revendication 1 ou 2, dans laquelle l'acide nucléique hétérologue code un produit thérapeutique et le médicament assure une expression prolongée du produit.

4. Utilisation selon l'une quelconque des revendications précédentes, dans laquelle la séquence d'acide nucléique hétérologue code une protéine sécrétable.

5. Utilisation selon la revendication 4, dans laquelle la protéine sécrétable est choisie parmi le facteur IX, l'apolipoprotéine E, l'interféron β, l'insuline, l'érythropoïétine, l'hormone de croissance et l'hormone parathyroïdienne.

6. Utilisation selon l'une quelconque des revendications précédentes, dans laquelle le rAAV comprend, en outre, des séquences terminales répétées inverses de l'AAV en 5' et 3' flanquant la séquence d'acide nucléique hétérologue.

7. Utilisation selon l'une quelconque des revendications précédentes, dans laquelle le rAAV comprend, de 5' à 3', des séquences terminales répétées inverses (ITRs) de l'AAV en 5', un promoteur hétérologue, la séquence d'acide nucléique hétérologue, une séquence de polyadénylation et des ITRs de l'AAV en 3'.

8. Utilisation selon la revendication 7, dans laquelle le promoteur hétérologue est un promoteur constitutif.

9. Utilisation selon la revendication 8, dans laquelle le promoteur constitutif est choisi parmi le promoteur précoce immédiat du cytomégalovirus et le promoteur LTR du virus du sarcome de Rous.

10. Utilisation selon la revendication 7, dans laquelle le promoteur hétérologue est un promoteur inductible.

11. Utilisation selon l'une quelconque des revendications précédentes, dans laquelle le muscle est choisi parmi les muscles squelettique, cardiaque et lisse.

12. Utilisation selon l'une quelconque des revendications précédentes, dans laquelle le médicament comprend au moins 10⁹ particules de rAAV.

13. Utilisation selon l'une quelconque des revendications précédentes, dans laquelle le médicament comprend 2,5 x 10¹⁰ à 5 x 10¹⁰ particules de rAAV.

14. Utilisation selon la revendication 13, dans laquelle le médicament est injecté dans les cellules musculaires à une dose de 2 x 10¹¹ génomes vecteurs de rAAV.

15. Utilisation selon l'une quelconque des revendications 1 à 6, dans laquelle la maladie chronique est l'hémophilie et le produit est le facteur IX.

16. Utilisation selon l'une quelconque des revendications 1 à 6, dans laquelle la maladie chronique est l'athérosclérose et le produit est l'apolipoprotéine E.


### SEQUENCE LISTING

(1) GENERAL INFORMATION:
   (i) APPLICANT: Trustees of the University of Pennsylvania
      Wilson, James M.
      Fisher, Krishna J.
   (ii) TITLE OF INVENTION: Method for Recombinant Adeno-Associated Virus-Directed Gene Therapy
   (iii) NUMBER OF SEQUENCES: 4
   (iv) CORRESPONDENCE ADDRESS:
      (A) ADDRESSEE: Howson and Howson
      (B) STREET: Spring House Corporate Cntr, PO Box 457
      (C) CITY: Spring House
      (D) STATE: Pennsylvania
      (E) COUNTRY: USA
      (F) ZIP: 19477
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER: WO
      (B) FILING DATE:
      (C) CLASSIFICATION:
   (vii) PRIOR APPLICATION DATA:
      (A) APPLICATION NUMBER: US 08/708,188
      (B) FILING DATE: 06-SEP-1996
   (vii) PRIOR APPLICATION DATA:
      (A) APPLICATION NUMBER: US 08/729,061
      (B) FILING DATE: 10-OCT-1996
   (viii) ATTORNEY/AGENT INFORMATION:
      (A) NAME: Kodroff, Cathy A.
      (B) REGISTRATION NUMBER: 33,980
      (C) REFERENCE/DOCKET NUMBER: GNVPN.019CIP2PCT
   (ix) TELECOMMUNICATION INFORMATION:
      (A) TELEPHONE: 215-540-9200
      (B) TELEFAX: 215-540-5818
(2) INFORMATION FOR SEQ ID NO:1:
   (i) SEQUENCE CHARACTERISTICS:
      (A) LENGTH: 10398 base pairs
      (B) TYPE: nucleic acid
      (C) STRANDEDNESS: double
      (D) TOPOLOGY: unknown
   (ii) MOLECULE TYPE: cDNA
   (xi) SEQUENCE DESCRIPTION: SEQ ID NO:1:
   GAATTCGCTA GCATCATCAA TAATATACCT TATTTTGGAT TGAAGCCAAT ATGATAATGA 60
   GGGGGTGGAG TTTGTGACGT GGCGCGGGGC GTGGGAACGG GGCGGGTGAC GTAGTAGTGT 120
   GGCGGAAGTG TGATGTTGCA AGTGTGGCGG AACACATGTA AGCGACGGAT GTGGCAAAAG 180
   TGACGTTTTT GGTGTGCGCC GGTGTACACA GGAAGTGACA ATTTTCGCGC GGTTTTAGGC 240
   GGATGTTGTA GTAAATTTGG GCGTAACCGA GTAAGATTTG GCCATTTTCG CGGGAAAACT 300
   GAATAAGAGG AAGTGAAATC TGAATAATTT TGTGTTACTC ATAGCGCGTA ATATTTGTCT 360
   AGGGAGATCT GCTGCGCGCT CGCTCGCTCA CTGAGGCCGC CCGGGCAAAG CCCGGGCGTC 420
   GGGCGACCTT TGGTCGCCCG GCCTCAGTGA GCGAGCGAGC GCGCAGAGAG GGAGTGGCCA 480
   ACTCCATCAC TAGGGGTTCC TTGTAGTTAA TGATTAACCC GCCATGCTAC TTATCTACAA 540
   TTCGAGCTTG CATGCCTGCA GGTCGTTACA TAACTTACGG TAAATGGCCC GCCTGGCTGA 600
   CCGCCCAACG ACCCCCGCCC ATTGACGTCA ATAATGACGT ATGTTCCCAT AGTAACGCCA 660
   ATAGGGACTT TCCATTGACG TCAATGGGTG GAGTATTTAC GGTAAACTGC CCACTTGGCA 720
   GTACATCAAG TGTATCATAT GCCAAGTACG CCCCCTATTG ACGTCAATGA CGGTAAATGG 780
   CCCGCCTGGC ATTATGCCCA GTACATGACC TTATGGGACT TTCCTACTTG GCAGTACATC 840
   TACGTATTAG TCATCGCTAT TACCATGGTG ATGCGGTTTT GGCAGTACAT CAATGGGCGT 900
   GGATAGCGGT TTGACTCACG GGGATTTCCA AGTCTCCACC CCATTGACGT CAATGGGAGT 960
   TTGTTTTGGC ACCAAAATCA ACGGGACTTT CCAAAATGTC GTAACAACTC CGCCCCATTG 1020
   ACGCAAATGG GCGGTAGGCG TGTACGGTGG GAGGTCTATA TAAGCAGAGC TCGTTTAGTG 1080
   AACCGTCAGA TCGCCTGGAG ACGCCATCCA CGCTGTTTTG ACCTCCATAG AAGACACCGG 1140
   GACCGATCCA GCCTCCGGAC TCTAGAGGAT CCGGTACTCG AGGAACTGAA AAACCAGAAA 1200
   GTTAACTGGT AAGTTTAGTC TTTTTGTCTT TTATTTCAGG TCCCGGATCC GGTGGTGGTG 1260
   CAAATCAAAG AACTGCTCCT CAGTGGATGT TGCCTTTACT TCTAGGCCTG TACGGAAGTG 1320
   TTACTTCTGC TCTAAAAGCT GCGGAATTGT ACCCGCGGCC GCAATTCCCG GGGATCGAAA 1380
   GAGCCTGCTA AAGCAAAAAA GAAGTCACCA TGTCGTTTAC TTTGACCAAC AAGAACGTGA 1440
   TTTTCGTTGC CGGTCTGGGA GGCATTGGTC TGGACACCAG CAAGGAGCTG CTCAAGCGCG 1500
   ATCCCGTCGT TTTACAACGT CGTGACTGGG AAAACCCTGG CGTTACCCAA CTTAATCGCC 1560
   TTGCAGCACA TCCCCCTTTC GCCAGCTGGC GTAATAGCGA AGAGGCCCGC ACCGATCGCC 1620
   CTTCCCAACA GTTGCGCAGC CTGAATGGCG AATGGCGCTT TGCCTGGTTT CCGGCACCAG 1680
   AAGCGGTGCC GGAAAGCTGG CTGGAGTGCG ATCTTCCTGA GGCCGATACT GTCGTCGTCC 1740
   CCTCAAACTG GCAGATGCAC GGTTACGATG CGCCCATCTA CACCAACGTA ACCTATCCCA 1800
   TTACGGTCAA TCCGCCGTTT GTTCCCACGG AGAATCCGAC GGGTTGTTAC TCGCTCACAT 1860
   TTAATGTTGA TGAAAGCTGG CTACAGGAAG GCCAGACGCG AATTATTTTT GATGGCGTTA    1920
   ACTCGGCGTT TCATCTGTGG TGCAACGGGC GCTGGGTCGG TTACGGCCAG GACAGTCGTT    1980
   TGCCGTCTGA ATTTGACCTG AGCGCATTTT TACGCGCCGG AGAAAACCGC CTCGCGGTGA    2040
   TGGTGCTGCG TTGGAGTGAC GGCAGTTATC TGGAAGATCA GGATATGTGG CGGATGAGCG    2100
   GCATTTTCCG TGACGTCTCG TTGCTGCATA AACCGACTAC ACAAATCAGC GATTTCCATG    2160
   TTGCCACTCG CTTTAATGAT GATTTCAGCC GCGCTGTACT GGAGGCTGAA GTTCAGATGT    2220
   GCGGCGAGTT GCGTGACTAC CTACGGGTAA CAGTTTCTTT ATGGCAGGGT GAAACGCAGG    2280
   TCGCCAGCGG CACCGCGCCT TTCGGCGGTG AAATTATCGA TGAGCGTGGT GGTTATGCCG    2340
   ATCGCGTCAC ACTACGTCTG AACGTCGAAA ACCCGAAACT GTGGAGCGCC GAAATCCCGA    2400
   ATCTCTATCG TGCGGTGGTT GAACTGCACA CCGCCGACGG CACGCTGATT GAAGCAGAAG    2460
   CCTGCGATGT CGGTTTCCGC GAGGTGCGGA TTGAAAATGG TCTGCTGCTG CTGAACGGCA    2520
   AGCCGTTGCT GATTCGAGGC GTTAACCGTC ACGAGCATCA TCCTCTGCAT GGTCAGGTCA    2580
   TGGATGAGCA GACGATGGTG CAGGATATCC TGCTGATGAA GCAGAACAAC TTTAACGCCG    2640
   TGCGCTGTTC GCATTATCCG AACCATCCGC TGTGGTACAC GCTGTGCGAC CGCTACGGCC    2700
   TGTATGTGGT GGATGAAGCC AATATTGAAA CCCACGGCAT GGTGCCAATG AATCGTCTGA    2760
   CCGATGATCC GCGCTGGCTA CCGGCGATGA GCGAACGCGT AACGCGAATG GTGCAGCGCG    2820
   ATCGTAATCA CCCGAGTGTG ATCATCTGGT CGCTGGGGAA TGAATCAGGC CACGGCGCTA    2880
   ATCACGACGC GCTGTATCGC TGGATCAAAT CTGTCGATCC TTCCCGCCCG GTGCAGTATG    2940
   AAGGCGGCGG AGCCGACACC ACGGCCACCG ATATTATTTG CCCGATGTAC GCGCGCGTGG    3000
   ATGAAGACCA GCCCTTCCCG GCTGTGCCGA AATGGTCCAT CAAAAAATGG CTTTCGCTAC    3060
   CTGGAGAGAC GCGCCCGCTG ATCCTTTGCG AATACGCCCA CGCGATGGGT AACAGTCTTG    3120
   GCGGTTTCGC TAAATACTGG CAGGCGTTTC GTCAGTATCC CCGTTTACAG GGCGGCTTCG    3180
   TCTGGGACTG GGTGGATCAG TCGCTGATTA AATATGATGA AAACGGCAAC CCGTGGTCGG    3240
   CTTACGGCGG TGATTTTGGC GATACGCCGA ACGATCGCCA GTTCTGTATG AACGGTCTGG    3300
   TCTTTGCCGA CCGCACGCCG CATCCAGCGC TGACGGAAGC AAAACACCAG CAGCAGTTTT    3360
   TCCAGTTCCG TTTATCCGGG CAAACCATCG AAGTGACCAG CGAATACCTG TTCCGTCATA    3420
   GCGATAACGA GCTCCTGCAC TGGATGGTGG CGCTGGATGG TAAGCCGCTG GCAAGCGGTG    3480
   AAGTGCCTCT GGATGTCGCT CCACAAGGTA AACAGTTGAT TGAACTGCCT GAACTACCGC    3540
   AGCCGGAGAG CGCCGGGCAA CTCTGGCTCA CAGTACGCGT AGTGCAACCG AACGCGACCG    3600
   CATGGTCAGA AGCCGGGCAC ATCAGCGCCT GGCAGCAGTG GCGTCTGGCG GAAAACCTCA    3660
   GTGTGACGCT CCCCGCCGCG TCCCACGCCA TCCCGCATCT GACCACCAGC GAAATGGATT    3720
   TTTGCATCGA GCTGGGTAAT AAGCGTTGGC AATTTAACCG CCAGTCAGGC TTTCTTTCAC    3780
   AGATGTGGAT TGGCGATAAA AAACAACTGC TGACGCCGCT GCGCGATCAG TTCACCCGTG 3840
   CACCGCTGGA TAACGACATT GGCGTAAGTG AAGCGACCCG CATTGACCCT AACGCCTGGG 3900
   TCGAACGCTG GAAGGCGGCG GGCCATTACC AGGCCGAAGC AGCGTTGTTG CAGTGCACGG 3960
   CAGATACACT TGCTGATGCG GTGCTGATTA CGACCGCTCA CGCGTGGCAG CATCAGGGGA 4020
   AAACCTTATT TATCAGCCGG AAAACCTACC GGATTGATGG TAGTGGTCAA ATGGCGATTA 4080
   CCGTTGATGT TGAAGTGGCG AGCGATACAC CGCATCCGGC GCGGATTGGC CTGAACTGCC 4140
   AGCTGGCGCA GGTAGCAGAG CGGGTAAACT GGCTCGGATT AGGGCCGCAA GAAAACTATC 4200
   CCGACCGCCT TACTGCCGCC TGTTTTGACC GCTGGGATCT GCCATTGTCA GACATGTATA 4260
   CCCCGTACGT CTTCCCGAGC GAAAACGGTC TGCGCTGCGG GACGCGCGAA TTGAATTATG 4320
   GCCCACACCA GTGGCGCGGC GACTTCCAGT TCAACATCAG CCGCTACAGT CAACAGCAAC 4380
   TGATGGAAAC CAGCCATCGC CATCTGCTGC ACGCGGAAGA AGGCACATGG CTGAATATCG 4440
   ACGGTTTCCA TATGGGGATT GGTGGCGACG ACTCCTGGAG CCCGTCAGTA TCGGCGGAAT 4500
   TACAGCTGAG CGCCGGTCGC TACCATTACC AGTTGGTCTG GTGTCAAAAA TAATAATAAC 4560
   CGGGCAGGCC ATGTCTGCCC GTATTTCGCG TAAGGAAATC CATTATGTAC TATTTAAAAA 4620
   ACACAAACTT TTGGATGTTC GGTTTATTCT TTTTCTTTTA CTTTTTTATC ATGGGAGCCT 4680
   ACTTCCCGTT TTTCCCGATT TGGCTACATG ACATCAACCA TATCAGCAAA AGTGATACGG 4740
   GTATTATTTT TGCCGCTATT TCTCTGTTCT CGCTATTATT CCAACCGCTG TTTGGTCTGC 4800
   TTTCTGACAA ACTCGGCCTC GACTCTAGGC GGCCGCGGGG ATCCAGACAT GATAAGATAC 4860
   ATTGATGAGT TTGGACAAAC CACAACTAGA ATGCAGTGAA AAAAATGCTT TATTTGTGAA 4920
   ATTTGTGATG CTATTGCTTT ATTTGTAACC ATTATAAGCT GCAATAAACA AGTTAACAAC 4980
   AACAATTGCA TTCATTTTAT GTTTCAGGTT CAGGGGGAGG TGTGGGAGGT TTTTTCGGAT 5040
   CCTCTAGAGT CGAGTAGATA AGTAGCATGG CGGGTTAATC ATTAACTACA AGGAACCCCT 5100
   AGTGATGGAG TTGGCCACTC CCTCTCTGCG CGCTCGCTCG CTCACTGAGG CCGGGCGACC 5160
   AAAGGTCGCC CGACGCCCGG GCTTTGCCCG GGCGGCCTCA GTGAGCGAGC GAGCGCGCAG 5220
   CAGATCTGGA AGGTGCTGAG GTACGATGAG ACCCGCACCA GGTGCAGACC CTGCGAGTGT 5280
   GGCGGTAAAC ATATTAGGAA CCAGCCTGTG ATGCTGGATG TGACCGAGGA GCTGAGGCCC 5340
   GATCACTTGG TGCTGGCCTG CACCCGCGCT GAGTTTGGCT CTAGCGATGA AGATACAGAT 5400
   TGAGGTACTG AAATGTGTGG GCGTGGCTTA AGGGTGGGAA AGAATATATA AGGTGGGGGT 5460
   CTTATGTAGT TTTGTATCTG TTTTGCAGCA GCCGCCGCCG CCATGAGCAC CAACTCGTTT 5520
   GATGGAAGCA TTGTGAGCTC ATATTTGACA ACGCGCATGC CCCCATGGGC CGGGGTGCGT 5580
   CAGAATGTGA TGGGCTCCAG CATTGATGGT CGCCCCGTCC TGCCCGCAAA CTCTACTACC 5640
   TTGACCTACG AGACCGTGTC TGGAACGCCG TTGGAGACTG CAGCCTCCGC CGCCGCTTCA 5700
   GCCGCTGCAG CCACCGCCCG CGGGATTGTG ACTGACTTTG CTTTCCTGAG CCCGCTTGCA 5760
   AGCAGTGCAG CTTCCCGTTC ATCCGCCCGC GATGACAAGT TGACGGCTCT TTTGGCACAA 5820
   TTGGATTCTT TGACCCGGGA ACTTAATGTC GTTTCTCAGC AGCTGTTGGA TCTGCGCCAG 5880
   CAGGTTTCTG CCCTGAAGGC TTCCTCCCCT CCCAATGCGG TTTAAAACAT AAATAAAAAA 5940
   CCAGACTCTG TTTGGATTTG GATCAAGCAA GTGTCTTGCT GTCTTTATTT AGGGGTTTTG 6000
   CGCGCGCGGT AGGCCCGGGA CCAGCGGTCT CGGTCGTTGA GGGTCCTGTG TATTTTTTCC 6060
   AGGACGTGGT AAAGGTGACT CTGGATGTTC AGATACATGG GCATAAGCCC GTCTCTGGGG 6120
   TGGAGGTAGC ACCACTGCAG AGCTTCATGC TGCGGGGTGG TGTTGTAGAT GATCCAGTCG 6180
   TAGCAGGAGC GCTGGGCGTG GTGCCTAAAA ATGTCTTTCA GTAGCAAGCT GATTGCCAGG 6240
   GGCAGGCCCT TGGTGTAAGT GTTTACAAAG CGGTTAAGCT GGGATGGGTG CATACGTGGG 6300
   GATATGAGAT GCATCTTGGA CTGTATTTTT AGGTTGGCTA TGTTCCCAGC CATATCCCTC 6360
   CGGGGATTCA TGTTGTGCAG AACCACCAGC ACAGTGTATC CGGTGCACTT GGGAAATTTG 6420
   TCATGTAGCT TAGAAGGAAA TGCGTGGAAG AACTTGGAGA CGCCCTTGTG ACCTCCAAGA 6480
   TTTTCCATGC ATTCGTCCAT AATGATGGCA ATGGGCCCAC GGGCGGCGGC CTGGGCGAAG 6540
   ATATTTCTGG GATCACTAAC GTCATAGTTG TGTTCCAGGA TGAGATCGTC ATAGGCCATT 6600
   TTTACAAAGC GCGGGCGGAG GGTGCCAGAC TGCGGTATAA TGGTTCCATC CGGCCCAGGG 6660
   GCGTAGTTAC CCTCACAGAT TTGCATTTCC CACGCTTTGA GTTCAGATGG GGGGATCATG 6720
   TCTACCTGCG GGGCGATGAA GAAAACGGTT TCCGGGGTAG GGGAGATCAG CTGGGAAGAA 6780
   AGCAGGTTCC TGAGCAGCTG CGACTTACCG CAGCCGGTGG GCCCGTAAAT CACACCTATT 6840
   ACCGGGTGCA ACTGGTAGTT AAGAGAGCTG CAGCTGCCGT CATCCCTGAG CAGGGGGGCC 6900
   ACTTCGTTAA GCATGTCCCT GACTCGCATG TTTTCCCTGA CCAAATCCGC CAGAAGGCGC 6960
   TCGCCGCCCA GCGATAGCAG TTCTTGCAAG GAAGCAAAGT TTTTCAACGG TTTGAGACCG 7020
   TCCGCCGTAG GCATGCTTTT GAGCGTTTGA CCAAGCAGTT CCAGGCGGTC CCACAGCTCG 7080
   GTCACCTGCT CTACGGCATC TCGATCCAGC ATATCTCCTC GTTTCGCGGG TTGGGGCGGC 7140
   TTTCGCTGTA CGGCAGTAGT CGGTGCTCGT CCAGACGGGC CAGGGTCATG TCTTTCCACG 7200
   GGCGCAGGGT CCTCGTCAGC GTAGTCTGGG TCACGGTGAA GGGGTGCGCT CCGGGCTGCG 7260
   CGCTGGCCAG GGTGCGCTTG AGGCTGGTCC TGCTGGTGCT GAAGCGCTGC CGGTCTTCGC 7320
   CCTGCGCGTC GGCCAGGTAG CATTTGACCA TGGTGTCATA GTCCAGCCCC TCCGCGGCGT 7380
   GGCCCTTGGC GCGCAGCTTG CCCTTGGAGG AGGCGCCGCA CGAGGGGCAG TGCAGACTTT 7440
   TGAGGGCGTA GAGCTTGGGC GCGAGAAATA CCGATTCCGG GGAGTAGGCA TCCGCGCCGC 7500
   AGGCCCCGCA GACGGTCTCG CATTCCACGA GCCAGGTGAG CTCTGGCCGT TCGGGGTCAA 7560
   AAACCAGGTT TCCCCCATGC TTTTTGATGC GTTTCTTACC TCTGGTTTCC ATGAGCCGGT 7620
   GTCCACGCTC GGTGACGAAA AGGCTGTCCG TGTCCCCGTA TACAGACTTG AGAGGCCTGT 7680
   CCTCGACCGA TGCCCTTGAG AGCCTTCAAC CCAGTCAGCT CCTTCCGGTG GGCGCGGGGC 7740
   ATGACTATCG TCGCCGCACT TATGACTGTC TTCTTTATCA TGCAACTCGT AGGACAGGTG 7800
   CCGGCAGCGC TCTGGGTCAT TTTCGGCGAG GACCGCTTTC GCTGGAGCGC GACGATGATC 7860
   GGCCTGTCGC TTGCGGTATT CGGAATCTTG CACGCCCTCG CTCAAGCCTT CGTCACTGGT 7920
   CCCGCCACCA AACGTTTCGG CGAGAAGCAG GCCATTATCG CCGGCATGGC GGCCGACGCG 7980
   CTGGGCTACG TCTTGCTGGC GTTCGCGACG CGAGGCTGGA TGGCCTTCCC CATTATGATT 8040
   CTTCTCGCTT CCGGCGGCAT CGGGATGCCC GCGTTGCAGG CCATGCTGTC CAGGCAGGTA 8100
   GATGACGACC ATCAGGGACA GCTTCAAGGA TCGCTCGCGG CTCTTACCAG CCTAACTTCG 8160
   ATCACTGGAC CGCTGATCGT CACGGCGATT TATGCCGCCT CGGCGAGCAC ATGGAACGGG 8220
   TTGGCATGGA TTGTAGGCGC CGCCCTATAC CTTGTCTGCC TCCCCGCGTT GCGTCGCGGT 8280
   GCATGGAGCC GGGCCACCTC GACCTGAATG GAAGCCGGCG GCACCTCGCT AACGGATTCA 8340
   CCACTCCAAG AATTGGAGCC AATCAATTCT TGCGGAGAAC TGTGAATGCG CAAACCAACC 8400
   CTTGGCAGAA CATATCCATC GCGTCCGCCA TCTCCAGCAG CCGCACGCGG CGCATCTCGG 8460
   GCAGCGTTGG GTCCTGGCCA CGGGTGCGCA TGATCGTGCT CCTGTCGTTG AGGACCCGGC 8520
   TAGGCTGGCG GGGTTGCCTT ACTGGTTAGC AGAATGAATC ACCGATACGC GAGCGAACGT 8580
   GAAGCGACTG CTGCTGCAAA ACGTCTGCGA CCTGAGCAAC AACATGAATG GTCTTCGGTT 8640
   TCCGTGTTTC GTAAAGTCTG GAAACGCGGA AGTCAGCGCC CTGCACCATT ATGTTCCGGA 8700
   TCTGCATCGC AGGATGCTGC TGGCTACCCT GTGGAACACC TACATCTGTA TTAACGAAGC 8760
   CTTTCTCAAT GCTCACGCTG TAGGTATCTC AGTTCGGTGT AGGTCGTTCG CTCCAAGCTG 8820
   GGCTGTGTGC ACGAACCCCC CGTTCAGCCC GACCGCTGCG CCTTATCCGG TAACTATCGT 8880
   CTTGAGTCCA ACCCGGTAAG ACACGACTTA TCGCCACTGG CAGCAGCCAC TGGTAACAGG 8940
   ATTAGCAGAG CGAGGTATGT AGGCGGTGCT ACAGAGTTCT TGAAGTGGTG GCCTAACTAC 9000
   GGCTACACTA GAAGGACAGT ATTTGGTATC TGCGCTCTGC TGAAGCCAGT TACCTTCGGA 9060
   AAAAGAGTTG GTAGCTCTTG ATCCGGCAAA CAAACCACCG CTGGTAGCGG TGGTTTTTTT 9120
   GTTTGCAAGC AGCAGATTAC GCGCAGAAAA AAAGGATCTC AAGAAGATCC TTTGATCTTT 9180
   TCTACGGGGT CTGACGCTCA GTGGAACGAA AACTCACGTT AAGGGATTTT GGTCATGAGA 9240
   TTATCAAAAA GGATCTTCAC CTAGATCCTT TTAAATTAAA AATGAAGTTT TAAATCAATC 9300
   TAAAGTATAT ATGAGTAAAC TTGGTCTGAC AGTTACCAAT GCTTAATCAG TGAGGCACCT 9360
   ATCTCAGCGA TCTGTCTATT TCGTTCATCC ATAGTTGCCT GACTCCCCGT CGTGTAGATA 9420
   ACTACGATAC GGGAGGGCTT ACCATCTGGC CCCAGTGCTG CAATGATACC GCGAGACCCA 9480
   CGCTCACCGG CTCCAGATTT ATCAGCAATA AACCAGCCAG CCGGAAGGGC CGAGCGCAGA 9540
   AGTGGTCCTG CAACTTTATC CGCCTCCATC CAGTCTATTA ATTGTTGCCG GGAAGCTAGA 9600
   GTAAGTAGTT CGCCAGTTAA TAGTTTGCGC AACGTTGTTG CCATTGCTGC AGGCATCGTG 9660
   GTGTCACGCT CGTCGTTTGG TATGGCTTCA TTCAGCTCCG GTTCCCAACG ATCAAGGCGA 9720
   GTTACATGAT CCCCCATGTT GTGCAAAAAA GCGGTTAGCT CCTTCGGTCC TCCGATCGTT 9780
   GTCAGAAGTA AGTTGGCCGC AGTGTTATCA CTCATGGTTA TGGCAGCACT GCATAATTCT 9840
   CTTACTGTCA TGCCATCCGT AAGATGCTTT TCTGTGACTG GTGAGTACTC AACCAAGTCA 9900
   TTCTGAGAAT AGTGTATGCG GCGACCGAGT TGCTCTTGCC CGGCGTCAAC ACGGGATAAT 9960
   ACCGCGCCAC ATAGCAGAAC TTTAAAAGTG CTCATCATTG GAAAACGTTC TTCGGGGCGA 10020
   AAACTCTCAA GGATCTTACC GCTGTTGAGA TCCAGTTCGA TGTAACCCAC TCGTGCACCC 10080
   AACTGATCTT CAGCATCTTT TACTTTCACC AGCGTTTCTG GGTGAGCAAA AACAGGAAGG 10140
   CAAAATGCCG CAAAAAAGGG AATAAGGGCG ACACGGAAAT GTTGAATACT CATACTCTTC 10200
   CTTTTTCAAT ATTATTGAAG CATTTATCAG GGTTATTGTC TCATGAGCGG ATACATATTT 10260
   GAATGTATTT AGAAAAATAA ACAAATAGGG GTTCCGCGCA CATTTCCCCG AAAAGTGCCA 10320
   CCTGACGTCT AAGAAACCAT TATTATCATG ACATTAACCT ATAAAAATAG GCGTATCACG 10380
   AGGCCCTTTC GTCTTCAA 10398
(2) INFORMATION FOR SEQ ID NO:2:
   (i) SEQUENCE CHARACTERISTICS:
      (A) LENGTH: 20 base pairs
      (B) TYPE: nucleic acid
      (C) STRANDEDNESS: single
      (D) TOPOLOGY: unknown
   (ii) MOLECULE TYPE: other nucleic acid
   (xi) SEQUENCE DESCRIPTION: SEQ ID NO:2:
      CATGGTAATA GCGATGACTA 20
(2) INFORMATION FOR SEQ ID NO:3:
   (i) SEQUENCE CHARACTERISTICS:
      (A) LENGTH: 20 base pairs
      (B) TYPE: nucleic acid
      (C) STRANDEDNESS: single
      (D) TOPOLOGY: unknown
   (ii) MOLECULE TYPE: other nucleic acid
   (xi) SEQUENCE DESCRIPTION: SEQ ID NO:3:
      GCTCTGCTTA TATAGACCTC 20
(2) INFORMATION FOR SEQ ID NO:4:
   (i) SEQUENCE CHARACTERISTICS:
      (A) LENGTH: 20 base pairs
      (B) TYPE: nucleic acid
      (C) STRANDEDNESS: single
      (D) TOPOLOGY: unknown
   (ii) MOLECULE TYPE: other nucleic acid
   (xi) SEQUENCE DESCRIPTION: SEQ ID NO:4:
      ATAAGCTGCA ATAAACAAGT 20